# FASCICULE DE BREVET EUROPEEN

(11) **EP 3 386 974 B1**
(45) Date de publication et mention de la délivrance du brevet: **05.02.2020**
(21) Numéro de dépôt: 16825810.1
(22) Date de dépôt: 09.12.2016
(51) Int. Cl.: C07D 401/14, C07F 15/00, C07F 3/00

(54) **AGENTS COMPLEXANTS HYDROSOLUBLES A BASE DE TRIAZAPYRIDONOPHANE ET COMPLEXES DE LANTHANIDE FLUORESCENTS CORRESPONDANTS**
WASSERLÖSLICHE TRIAZAPYRIDINOPHANBASIERTE KOMPLEXBILDNER UND ENTSPRECHENDE FLUORESZIERENDE LANTHANIDKOMPLEXE
WATER-SOLUBLE TRIAZAPYRIDINOPHANE-BASED COMPLEXING AGENTS AND CORRESPONDING FLUORESCENT LANTHANIDE COMPLEXES

(30) Priorité: 09.12.2015 FR 1562068
(43) Date de publication de la demande: 17.10.2018
(73) Titulaire: CISBIO BIOASSAYS, 30200 Codolet (FR); Université Paul Sabatier, 31062 Toulouse Cedex 9 (FR); Centre National de la Recherche Scientifique, 75016 Paris (FR)
(72) Inventeur: LAMARQUE, Laurent, 30290 Saint-Victor Lacoste (FR); PICARD, Claude, 31320 Auzeville-Tolosane (FR); GALAUP, Chantal, 31320 Rebigue (FR); LEYGUE, Nadine, 31520 Ramonville Saint Agne (FR); ZWIER, Jurriaan, 30650 Rochefort Du Gard (FR); BOURRIER, Emmanuel, 30200 Bagnols-sur-Ceze (FR)
(74) Mandataire: Cabinet Beau de Loménie
(86) Numéro de dépôt international: PCT/FR2016/053296
(87) Numéro de publication internationale: WO 2017/098180

(56) Documents cités:
- WO-A1-94/26276
- WO-A1-2014/111661
- CASTRO ET AL.: "Lanthanide(III) complexation with an amide derived pyridinophane", INORG. CHEM., vol. 54, no. 4, 23 janvier 2015 (2015-01-23), pages 1671-1683, XP002754980, cité dans la demande

## Description

La présente invention a pour objet de nouveaux agents complexant hydrosolubles basés sur des macrocycles de type triazapyridinophane, des complexes de lanthanides fluorescents obtenus à partir de ces agents complexants, et l'utilisation de ces complexes de lanthanides fluorescents pour marquer des molécules et les détecter par des techniques de fluorescence en temps résolu.

### Etat de la technique

Les complexes d'europium et de terbium sont désormais acceptés par l'ensemble de la communauté scientifique pour être des sondes fluorescentes de choix (Inorganic Chemistry 2014, 53, 1854, Chemical Review 2010, 110, 2729). En effet grâce à leurs propriétés photo-physiques très spécifiques, ces molécules ont été largement utilisées dans l'industrie et étudiés par les laboratoires universitaires dans le domaine des sciences de la vie. Ces composés fluorescents sont particulièrement appropriés pour être utilisés en conjonction avec des fluorophores compatibles pour effectuer des mesures de FRET (acronyme de l'expression anglaise « Förster Resonnance Energy Transfer »), dont l'application pour étudier les interactions entre biomolécules est exploitée de manière commerciale par plusieurs sociétés, dont Cisbio Bioassays et sa gamme de produits HTRF®. La durée de vie d'émission relativement longue des complexes de lanthanides permet également d'effectuer des mesures de fluorescence en temps résolu, c'est-à-dire avec un délai après excitation des fluorophores, ce qui permet de limiter les interférences de fluorescence dues au milieu de mesure. Cette dernière caractéristique est d'autant plus utile que le milieu de mesure se rapproche d'un milieu biologique, qui comprend de nombreuses protéines dont la fluorescence pourrait interférer avec celle des composés étudiés.

De nombreux complexes de lanthanides ont été divulgués et certains sont exploités de manière commerciale : on peut citer en particulier les cryptates macropolycycliques de lanthanides (EP 0 180 492, EP 0 321 353, EP 0 601 113 , WO 2001/96877, WO2008/063721), les complexes de lanthanides comportant un motif dérivé de la coumarine lié à un motif diéthylènetriamine penta-acide (US 5,622,821), et ceux comprenant des dérivés de pyridine (US 4,920,195, US 4,761,481), de bipyridine (US 5,216,134), ou de terpyridine (US 4,859,777, US 5,202,423, US 5,324,825).

Le brevet US 5,385,893 décrit des dérivés de triazapyridinophanes dont les trois azotes sont substitués par des groupes acides (carboxyliques ou phosphoniques), et des complexes de ces composés avec un atome de gadolinium, de manganèse ou de fer. Les complexes décrits ne sont pas fluorescents et peuvent être utilisés comme agents de contraste. Les triazapyridinophanes selon US 5,385,893 sont très peu adaptés à la préparation de complexes fluorescents du fait de la taille de la cage macrocyclique qui permet les échanges de molécules d'eau assez facilement. La synthèse de ce macrocycle triazapyridinophane a été réalisée avec un rendement de 2%. L'obtention de ce composé n'est pas directe et passe obligatoirement par l'utilisation d'un intermédiaire dont les trois atomes d'azote appartenant au macrocycle sont protégés par des groupements tosyles. Ces derniers sont éliminés en fin de synthèse dans des conditions drastiques.

Un peu plus tard l'amélioration de la synthèse a été rapportée par Lee et al. (Journal Organic Chemistry 1996, 61, 8304) qui obtiennent de meilleurs rendements. Le composé macrocyclique est obtenu selon une synthèse convergente comportant 8 étapes dont la dernière consiste à déprotéger les groupements tosyles, là encore en milieu fortement acide. Ces macrocycles ne sont pas utilisés comme ligands complexants.

En 2008 Nolan et al. ont tenté de reproduire le procédé de synthèse décrit par Lee mais ont échoué, ce qui les a conduit à développer une nouvelle stratégie de synthèse convergente (Tetrahedron Letters 2008, 49, 1993). L'étape finale de déprotection des tosyles est réalisée encore une fois en milieu très acide.

Récemment les travaux de Castro et al. sur les systèmes macrocycliques de type triazapyridinophane (Inorganic Chemistry 2015, 54, 1671 et Inorganic Chemistry 2013, 52, 6062 ont permis de caractériser les complexes de lanthanides correspondants par diffraction des rayons X. Cependant la méthode de synthèse utilisée est la même que celle décrite précédemment par Lee et al.

Les macrocycles triazapyridinophanes de l'art antérieur ne sont pas adaptés à une utilisation pour des dosages biologiques en FRET :
- Les macrocycles triazapyridinophanes complexés avec un atome d'europium ou de terbium décrits précédemment présentent des longueurs d'onde d'absorption optimales à 267 nm ce qui est rédhibitoire pour une utilisation comme composés fluorescents donneurs pour des mesures de FRET. En effet, d'une part les sources laser utilisées dans les dosages biologiques pour exciter les fluorophores donneurs émettent à différentes longueur d'ondes : 337 nm (laser à azote), 355 nm (laser Nd : YAG), 349 nm (Nd : YLF) et d'autre part les lampes flash qui permettent l'excitation entre 310 et 350 nm : les composés de l'art antérieur, absorbant de manière optimale à 267 nm, ne peuvent pas être excités par ces dispositifs.
- Les macrocycles triazapyridinophanes de l'art antérieur sont relativement hydrophobes, ce qui est un problème pour leur utilisation dans des dosages biologiques en milieu aqueux, et peut entraîner un important signal non-spécifique dans les mesures de FRET. De plus les complexes peu solubles dans les milieux aqueux ne sont tout simplement pas utilisables.
- Les méthodes de synthèse de ces macrocycles comprennent plusieurs étapes dont la dernière nécessite des conditions expérimentales très drastiques pour la libération des fonctions amines secondaires protégées par des groupements tosyles, à savoir plusieurs heures de réaction dans de l'acide sulfurique concentré à 120°C. Ceci est un véritable obstacle pour la synthèse de molécules plus élaborées utilisables comme fluorophore dans des dosages biologiques de type FRET, puisque ces conditions ne sont pas compatibles avec l'ajout de fonctions sensibles à ces conditions.
- Il est par ailleurs important de noter que les méthodes de synthèse de triazapyridinophanes décrites dans la littérature ne permettent d'accéder qu'à des composés de symétrie C₃ et donc ne permettent pas de différencier les substituants portés par les trois noyaux pyridines pour obtenir des composes adaptés à une utilisation comme fluorophore dans des dosages biologique en FRET.

Finalement, les macrocycles triazapyridinophanes de l'art antérieur ne sont non seulement pas adaptés à une utilisation comme fluorophore dans des dosages biologiques de type FRET, du fait de leur propriétés spectroscopique et de leur hydrophobie, mais en plus les méthodes décrites pour leurs synthèses rendent impossible toute adaptation de ces molécules à cette application (molécules de symétrie C₃, conditions trop drastiques de déprotection des amines).

Il existe donc un besoin pour des macrocycles triazapyridinophanes et des complexes de lanthanides correspondants utilisables dans des dosages biologiques de type FRET.

La présente invention vise à pallier aux inconvénients des composés de l'art antérieur, et à fournir des complexes de lanthanides fluorescents présentant une meilleure brillance que les composés de l'art antérieur lorsqu'ils sont excités entre 310 et 350 nm, si possible une bonne solubilité en milieu aqueux, un spectre d'émission adapté à leur utilisation dans des expériences de FRET, ainsi qu'une bonne praticité pour le marquage de biomolécules.

### Description de l'invention

Les inventeurs ont dépassé le préjugé technique selon lequel les triazapyridinophanes sont très peu adaptés à la préparation de complexes fluorescents du fait de la taille de la cage macrocyclique qui permet les échanges de molécules d'eau assez facilement. Les problèmes mentionnés précédemment ont été résolus grâce à l'élaboration d'un protocole de synthèse permettant la préparation d'agents complexants constitués d'un macrocycle triazapyridinophane, dont les pyridines sont substituées par des groupements paraméthoxyphénylacétylènes ou 2,4,6-triméthoxyphényles, ces groupements portant eux-mêmes des groupes susceptibles d'augmenter l'hydrosolubilité des macrocycles, et éventuellement un groupement permettant la conjugaison des macrocycles avec d'autres molécules, notamment des molécules biologiques que l'on souhaite marquer avec un composé fluorescent.

L'invention a donc pour objet des agents complexants basés sur un macrocycle triazapyridinophane, des complexes de lanthanides fluorescents constitués d'un atome de lanthanide complexé par un agent complexant selon l'invention, et des molécules organiques d'intérêts marquées par un complexe selon l'invention.

### Agents complexants

Les agents complexants selon l'invention sont les composés de formule (I) : 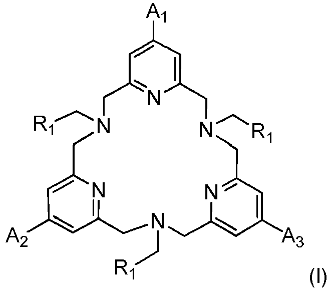 dans laquelle :
- les groupes R₁ sont identiques et sont choisis parmi : -CO₂H, -PO(OH)R, R étant choisi parmi les groupes: phényle, phényle substitué par un groupe -SO₃H, de préférence en position ortho ou para, benzyle, méthyle, éthyle, propyle, n-butyle, *sec*-butyle, isobutyle, *tert*-butyle;
- les groupes A₁, A₂ sont identiques ou différents et sont choisis parmi : un groupe de formule -L₁-E ; un groupe de formule (II) ou (II') ;
- le groupe A₃ est choisi parmi : un groupe de formule -O-L₃-G, un groupe de formule (II), (II'), (III) ou (III') ; 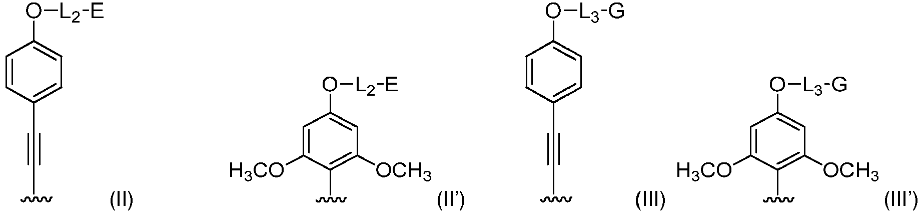
- L_{1,} L₂ et L₃ sont identiques ou différents et représentent un groupe de liaison divalent tel que défini dans les revendications ;
- E est un groupe qui rend l'agent complexant hydrosoluble, choisi parmi : -SO₃H, -PO(OH)₂,-CO₂H, -N⁺Alk₁Alk₂Alk₃, un résidu carbohydrate; une sulfobétaïne ; un groupe PEG ;
- G est tel que défini dans les revendications ;
- Alk₁, Alk₂, Alk₃, qui peuvent être identiques ou différents, représentent un (C₁-C₆)alkyle.

Par carbohydrate, on entend : un résidu glucose sous forme cyclique ou linéaire, ou bien un groupe de formule -(CHOHₖCH₂OH, k étant un nombre entier allant de 3 à 12, de préférence, égal à 4.

Par sulfobétaïne on entend un groupe choisi parmi : 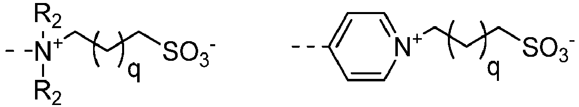 avec R₂ qui représente un groupe alkyle de 1 à 6 atomes de carbone, et de préférence un méthyle ou éthyle, et q qui est égal à 1, 2, 3, 4, 5 ou 6, et de préférence qui est égal à 1 ou 2, la sulfobétaïne de formule -(CH₃)₂N⁺-(CH₂)₃-SO₃⁻ étant préférée.

En fonction du pH, les groupes -SO₃H, -CO₂H et -PO(OH)₂ sont sous forme déprotonée ou pas. Ces groupes désignent donc dans la suite du texte également les groupes -SO₃⁻, -CO₂⁻ et -PO(OH)O⁻, et vice-versa.

Par groupe PEG on entend un groupe polyéthylène glycol de formule -CH₂-(CH₂OCH₂)_{y}-CH₂OCH₃, y étant un nombre entier allant de 1 à 5.

Les groupes (II), (II'), (III) et (III') contribuent à la formation d'antennes qui contribuent aux propriétés spectroscopiques des fluorophores. Les groupes -L₁-E, (II) et (II') portent des groupements E qui rendent les agents complexants hydrosolubles, et les groupes -O-L₃-G, (III) et (III') portent un groupe réactif G permettant de conjuguer les complexes fluorescents selon l'invention avec une molécule d'intérêt que l'on souhaite marquer.

Selon l'invention, les antennes comportent des groupes 2,4,6-méthoxyphényles (II'), (III') ou paraméthoxyphénylacétylènes (II), (III), ces derniers étant préférés. Par ailleurs les composés selon l'invention peuvent comporter 1, 2 ou 3 antennes, et les agents complexants selon l'invention peuvent ainsi être subdivisés selon les sous-familles préférées suivantes, à base d'antennes paraméthoxyphénylacétylènes :
- Agents complexants à 3 antennes, comportant 2 groupes solubilisants et un groupe réactif, correspondants aux composés de formule (I) dans lesquels A₁ et A₂ sont identiques et sont des groupes de formule (II) et A₃ est un groupe de formule (III).
- Agents complexants à 2 antennes comportant 2 groupes solubilisants et un groupe réactif, correspondants aux composés de formule (I) dans lesquels A₁ est un groupe de formule (II), A₂ est un groupe -L₁-E et A₃ est un groupe de formule (III).
- Agents complexants à 2 antennes comportant 2 groupes solubilisants et un groupe réactif, correspondants aux composés de formule (I) dans lesquels A₁ et A₂ sont identiques et sont des groupes de formule (II), et A₃ est un groupe de formule -O-L₃-G.
- Agents complexants à 1 antenne, comportant 2 groupes solubilisants et un groupe réactif, correspondants aux composés de formule (I) dans lesquels A₁ et A₂ sont identiques et sont des groupes de formule -L₁-E et A₃ est un groupe de formule (III).

On distingue les mêmes sous-familles à base d'antennes paraméthoxyphényles en remplaçant les groupes de formule (II) et (III) par les groupes de formule (II') et (III'), respectivement, à savoir :
- Agents complexants à 3 antennes, comportant 2 groupes solubilisants et un groupe réactif, correspondants aux composés de formule (I) dans lesquels A₁ et A₂ sont identiques et sont des groupes de formule (II') et A₃ est un groupe de formule (III').
- Agents complexants à 2 antennes comportant 2 groupes solubilisants et un groupe réactif, correspondants aux composés de formule (I) dans lesquels A₁ est un groupe de formule (II'), A₂ est un groupe -L₁-E et A₃ est un groupe de formule (III').
- Agents complexants à 2 antennes comportant 2 groupes solubilisants et un groupe réactif, correspondants aux composés de formule (I) dans lesquels A₁ et A₂ sont identiques et sont des groupes de formule (II'), et A₃ est un groupe de formule -O-L₃-G.
- Agents complexants à 1 antenne, comportant 2 groupes solubilisants et un groupe réactif, correspondants aux composés de formule (I) dans lesquels A₁ et A₂ sont identiques et sont des groupes de formule -L₁-E et A₃ est un groupe de formule (III').

Pour chacune de ces sous-familles, les groupes R₁ qui participent à la complexation du lanthanide peuvent être soit un carboxylate -CO₂H soit un phosphinate -PO(OH)R tel que défini ci-dessus (et de préférence dans ce dernier cas un méthylphosphinate).

Pour chacune de ces sous-familles, les composés dont les groupes E sont soit des groupes -SO₃⁻, soit un résidu glucose, soit un ammonium -N⁺(CH₃)₃ ou soit une sulfobétaïne de formule ci-dessous sont préférés

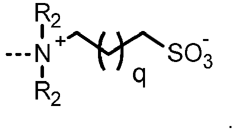

Les composés dont les groupes E sont soit des groupes -SO₃⁻, soit un résidu glucose, ou soit une sulfobétaïne de formule ci-dessus, sont particulièrement préférés.

Le groupe réactif G porté par un bras d'espacement L₃, permet de coupler les composés selon l'invention avec une espèce que l'on souhaite rendre fluorescente, par exemple une molécule organique, un peptide ou une protéine. Les techniques de conjugaison de deux molécules organiques sont basées sur l'utilisation de groupes réactifs et relèvent des connaissances générales de l'homme du métier. Ces techniques classiques sont décrites par exemple dans Bioconjugate Techniques, G.T. Hermanson, AcademicPress, Second Edition 2008, p. 169-211.

Typiquement, le groupe réactif est un groupe électrophile, nucléophile, diène ou diénophile qui peut former une liaison covalente lorsqu'il est mis en présence d'un groupe nucléophile, électrophile, diénophile ou diène approprié, respectivement. La réaction de conjugaison entre un composé selon l'invention comportant un groupe réactif et une molécule organique, un peptide ou une protéine portant un groupe fonctionnel entraîne la formation d'une liaison covalente comportant un ou plusieurs atomes du groupe réactif.

Le groupement réactif G est un groupe dérivé d'un des composés ci-après : un acrylamide, une amine activée (par exemple une cadavérine ou une éthylènediamine), un ester activé, un aldéhyde, un halogénure d'alkyle, un anhydride, une aniline, un azide, une aziridine, un acide carboxylique, un diazoalcane, un haloacétamide, une halotriazine, telle que la monochlorotriazine, la dichlorotriazine, une hydrazine (y compris les hydrazides), un imido ester, un isocyanate, un isothiocyanate, un maléimide, un halogénure de sulfonyle, un thiol, une cétone, une amine, un halogénure d'acide, un ester de succinimidyle, un ester d'hydroxysuccinimidyle, un ester d'hydroxysulfosuccinimidyle, un azidonitrophényle, un azidophényle, un glyoxal, une triazine, un groupe acétylénique, et les groupes de formule : 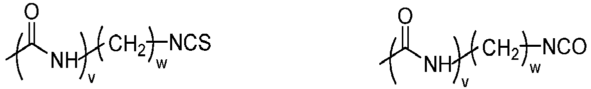 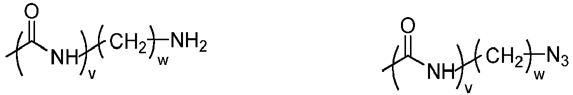 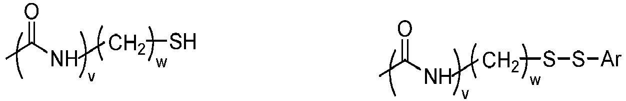 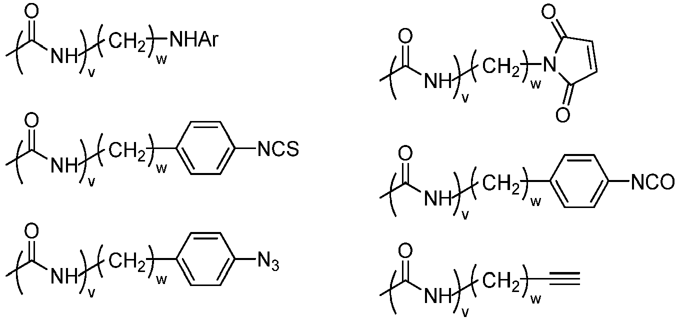 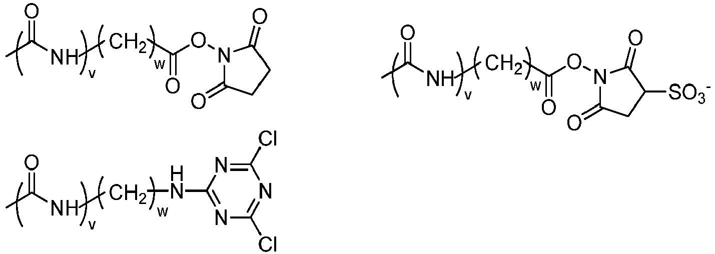 dans lesquels w varie de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène.

De manière préférée, le groupe réactif G est un acide carboxylique, une amine, de préférence une amine aliphatique (éventuellement protégée sous forme -NHBoc), un ester de succinimidyle, un haloacétamide, une hydrazine, un isothiocyanate, un groupe maléimide, ou un acide carboxylique (éventuellement protégé sous la forme d'un groupe -CO₂Me, -CO₂*t*Bu). Dans ce dernier cas, l'acide devra être activé sous forme d'ester pour pouvoir réagir avec une espèce nucléophile

Une molécule organique, un peptide ou une protéine susceptible d'être marquée par un composé selon l'invention comprendra donc un groupe fonctionnel avec lequel réagira le groupe réactif du complexe de lanthanide ou de l'agent complexant. Par exemple, la molécule organique, la protéine ou le peptide comporte un des groupes suivants : amine, amide, thiol, alcool, aldéhyde, cétone, hydrazine, hydroxylamine, amine secondaire, halogénure, époxyde, ester (carboxylate d'alkyle), acide carboxylique, des groupes comportant des doubles liaisons ou une combinaison de ces groupes fonctionnels. Les groupes amines ou thiols présents naturellement sur les protéines sont souvent utilisés pour procéder au marquage de ces molécules.

Le groupe réactif et les groupes solubilisants E sont liés à l'agent complexant *via* un bras d'espacement (L₁, L₂, L₃) constitué par un radical organique bivalent, choisi parmi
- un groupe alkylène linéaire ou ramifié en C₁-C₂₀, contenant éventuellement une ou plusieurs doubles ou triples liaisons;
- un groupe cycloalkylène en C₅-C₈ ou un groupe arylène en C₆-C₁₄,
   lesdits groupes alkylène, cycloalkylène ou arylène contenant éventuellement un ou plusieurs hétéroatomes, tels que l'oxygène, l'azote, le soufre, le phosphore ou un ou plusieurs groupe(s) carbamoyle ou carboxamido, et lesdits groupes alkylène, cycloalkylène ou arylène étant éventuellement substitués par des groupes alkyle en C₁-C₈, aryle en C₆-C₁₄, sulfonate ou oxo.
- un groupe choisi parmi les groupes divalents de formules suivantes : 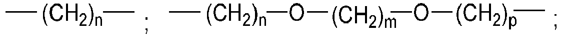 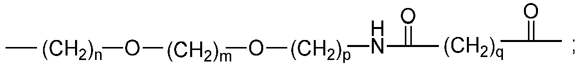 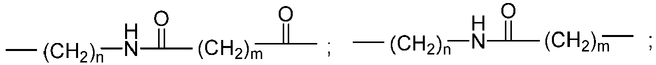 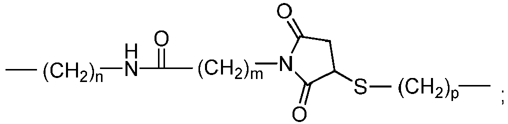 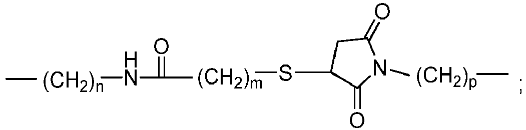 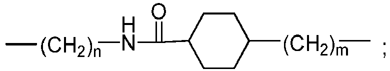 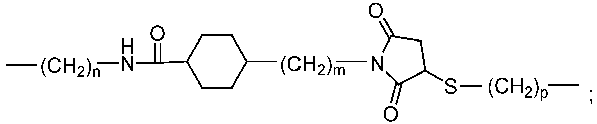 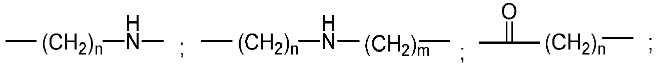 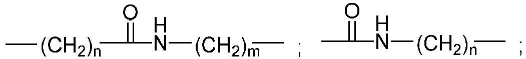
dans lesquels n, m, p, q sont des nombres entiers de 1 à 16, de préférence de 1 à 5.

De manière préférée, le groupe -L₃G est constitué d'un groupement réactif G choisi parmi : un acide carboxylique (éventuellement protégé sous la forme d'un groupe -CO₂Me, -CO₂*t*Bu), une amine, de préférence une amine aliphatique (éventuellement protégée sous forme -NHBoc), un ester de succinimidyle, un haloacétamide, une hydrazine, un isothiocyanate, un groupe maléimide, et d'un bras d'espacement L₃ constitué d'une chaîne alkylène comprenant de 1 à 5 atomes de carbone. De manière encore plus préférée, le groupe -L₃G est une amine portée par une chaîne alkylène comprenant de 1 à 5 atomes de carbone.

En ce qui concerne L₁, le groupe divalent suivant est préféré : 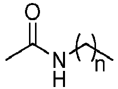 où n est un nombre entier de 1 à 16, de préférence de 1 à 5

En ce qui concerne L₂, le groupe divalent suivant est préféré : 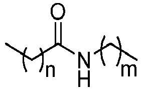 où n et m sont des nombres entiers de 1 à 16, de préférence de 1 à 5

En ce qui concerne L₃, le groupe divalent suivant est préféré :

-(CH₂)ₙ-

où n est un nombre entier de 1 à 16, de préférence de 1 à 5

Les sous-familles dans lesquelles, à la fois,
L₁ est le groupe divalent de formule : 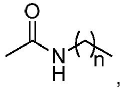 et
L₂ est le groupe divalent de formule 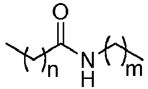 et
L₃ est le groupe divalent de formule 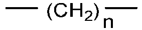 sont également préférées.

### Complexes

L'invention concerne également les complexes de lanthanides fluorescents constitués d'un atome de lanthanide complexé par un agent complexant tel que décrit ci-dessus. De préférence, le lanthanide est Tb³⁺, Eu³⁺ ou Sm³⁺ et de manière encore plus préférée Eu³⁺.

Ces complexes sont préparés en mettant en contact les agents complexants selon l'invention et un sel de lanthanide. Ainsi la réaction entre un équivalent d'agent complexant et 1 à 5 équivalents de sel de lanthanide (europium, terbium ou samarium sous forme par exemple de chlorures) dans un milieu aqueux à pH 6 conduit après plusieurs heures voire quelques jours de réaction à température ambiante au complexe correspondant.

Comme indiqué précédemment, les complexes fluorescents obtenus présentent d'excellentes propriétés photo-physiques, en particulier en ce qui concerne leur rendement quantique, la durée de vie de leur luminescence et leur spectre d'excitation qui est très bien adapté à une excitation laser à environ 337 nm. De plus la répartition des bandes de leurs spectres d'émission est centrée autour de 620 nm conférant ainsi aux complexes des propriétés exceptionnelles et très favorables dans une utilisation de FRET avec des accepteurs de type cyanine ou allophycocyanine (telle que la XL665 commercialisée par Cisbio Bioassays).

De manière avantageuse, l'invention concerne les complexes suivants : 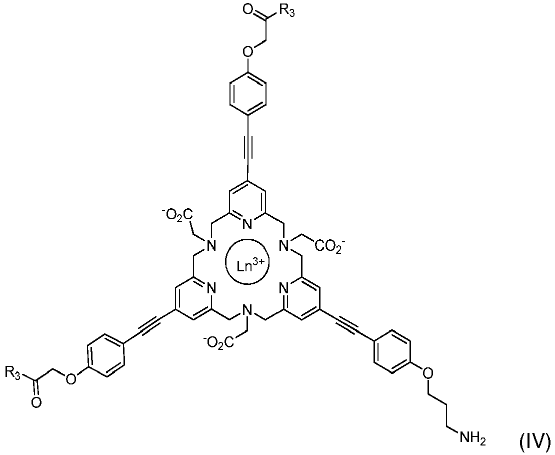 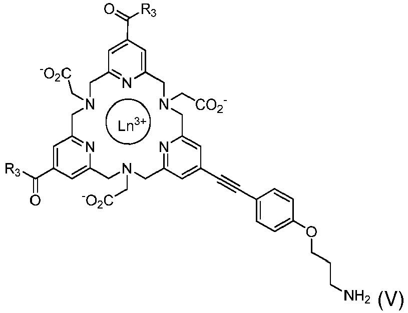 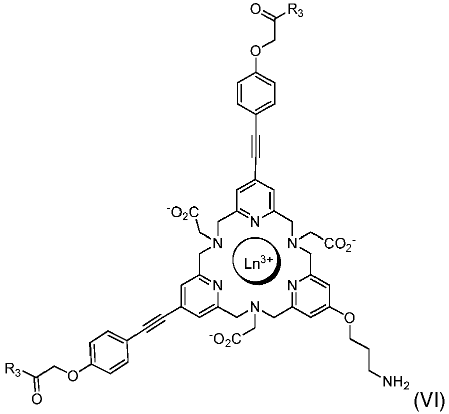 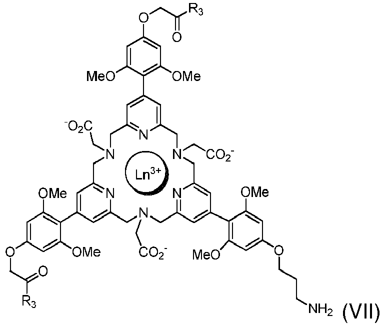 dans lesquelles :
Ln³⁺ est choisi parmi : Eu³⁺, Tb³⁺, Sm³⁺ ;
R₃ est choisi parmi les groupes suivants : OH ;

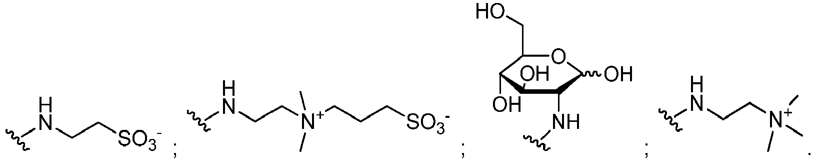

### Conjugués

Les agents complexants et complexes selon l'invention comportant un groupe réactif sont particulièrement adaptés au marquage de molécules organiques ou biologiques comportant un groupe fonctionnel susceptible de réagir avec le groupe réactif pour former une liaison covalente. Ainsi l'invention concerne aussi l'utilisation de complexes pour le marquage de molécules biologiques (protéines, anticorps, enzymes, hormones etc...).

L'invention concerne également les molécules marquées par un complexe selon l'invention. Toutes les molécules organiques ou biologiques peuvent être conjuguées avec un complexe selon l'invention si elles possèdent un groupe fonctionnel susceptible de réagir avec le groupe réactif. En particulier, les conjugués selon l'invention comportent un complexe selon l'invention et une molécule choisie parmi : un acide aminé, un peptide, une protéine, un anticorps, un sucre, une chaîne glucidique, un nucléoside, un nucléotide, un oligonucléotide, un substrat d'enzyme (en particulier un substrat d'enzyme suicide telle qu'une benzylguanine ou une benzylcytosine (substrats des enzymes commercialisées sous les dénominations SNAP-tag® et CLIP-tag®), un chloroalcane (substrat de l'enzyme commercialisée sous la dénomination Halo-tag), le coenzyme A (substrat de l'enzyme commercialisée sous le nom ACP-tag ou MCP-tag). Les conjugués de l'invention sont obtenus par mise en contact d'un complexe de lanthanide selon l'invention, comprenant un groupe réactif G, avec une molécule d'intérêt.

### Synthèses

La préparation des agents complexants et des complexes de lanthanides selon l'invention est décrite de manière schématique ci-après, et de manière plus détaillée dans la partie expérimentale.

### Synthèse systèmes tri-antennes (pyridyl-acétylène-4-O-phényl)

La synthèse des complexes comportant 3 antennes, 2 groupes solubilisants et 1 groupe réactif est décrite dans les schémas 1-4 et s'appuie sur une synthèse convergente. Les composés comportant d'autres groupes solubilisants / groupes réactifs peuvent être préparés de manière similaire à partir de l'intermédiaire **24.**

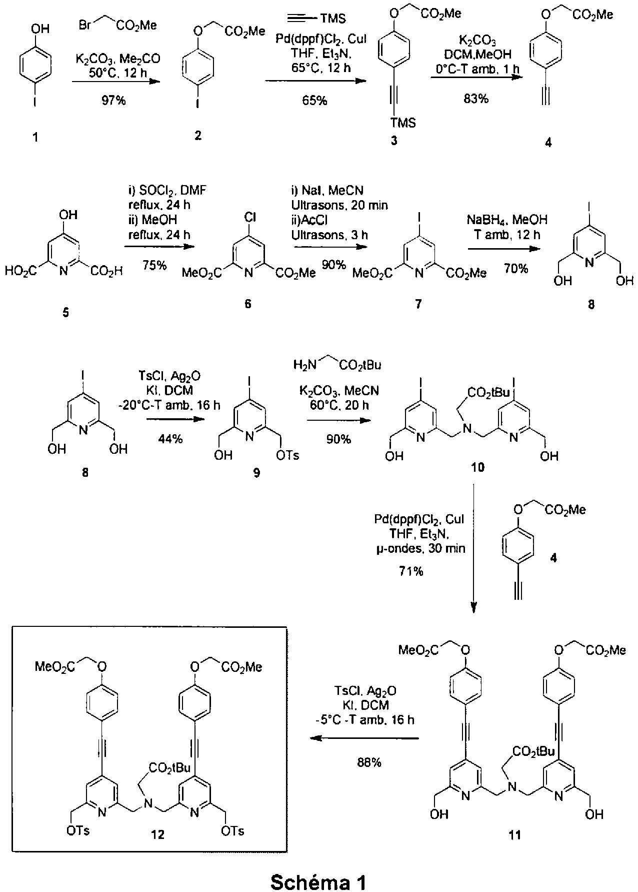

L'introduction de la fonction ester permettant ultérieurement la fixation d'une fonction solubilisante de type sulfonate, sulfo-bétaïne, ammonium ou dérivés de sucres est réalisée par une réaction d'alkylation sur le 4-iodo-phénol. Une réaction de couplage de type Sonogashira entre le dérivé **2** et le triméthylsilyl acétylène permet d'obtenir le composé **3.** Le groupe protecteur (triméthylsilyl) est supprimé en utilisant un mélange de solvants méthanol-dichlorométhane en présence de carbonate de potassium. Ces conditions permettent d'épargner la fonction ester méthylique qui est relativement sensible à l'hydrolyse. Le diol **8** est obtenu à partir de l'acide chélidamique en 3 étapes comme décrit dans la littérature (Tetrahedron 2005, 61, 1755 ; Tetrahedron 2008, 64, 399. European Journal of Organic Chemistry 2002, 21, 3680). La réaction de monotosylation est réalisée en présence de sels d'argent. Ces conditions ont été développées sur l'analogue non-halogéné. (Tetrahedron 2004, 49, 11117; et Angewandte Chemie, International Edition 2014, 53, 5872). La réaction de condensation permet d'obtenir le motif di-pyridinyle 10 sur lequel est réalisée une double réaction de Sonogashira conduisant au squelette du premier synthon clé. Les fonctions diols sont activées par l'introduction des groupements tosyles dans des conditions assez similaires à celles de la monotosylation.

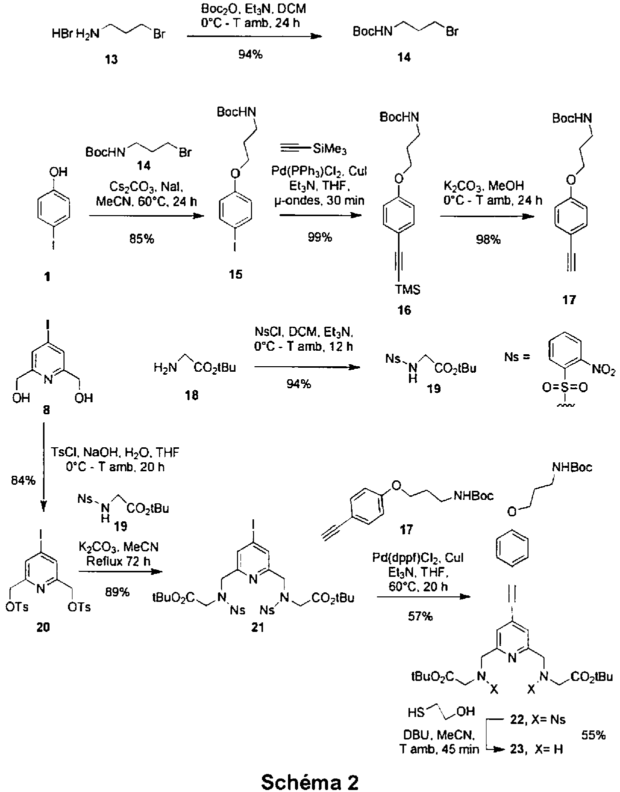

La synthèse du deuxième synthon clé **23** est décrite dans le schéma 2. Ce synthon portera une fonction amine masquée (groupement protecteur Boc) permettant par exemple une réaction de bioconjugaison avec un anticorps ou une biomolécule. Le composé **17** a été préparé selon les procédures décrites dans la littérature (WO 2014/111661). Le diol **8** est activé sous forme de ditosylate puis condensé avec le composé **19** conduisant au composé **21** avec un bon rendement. La réaction de Sonogashira suivie de la déprotection du groupement nosyle permet l'obtention du synthon **23.**

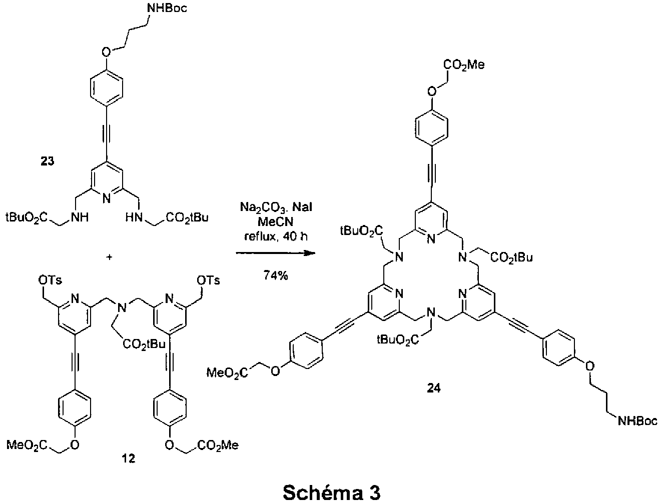

L'étape la plus délicate est représentée dans le schéma 3. Il s'agit de l'étape de macrocyclisation qui après optimisation a conduit au ligand **24** avec un rendement très convenable. Cet intermédiaire constitue une plateforme pour réaliser des séries de complexes de lanthanides (europium, terbium et samarium) portant différents groupes solubilisants comme décrit dans le schéma 4.

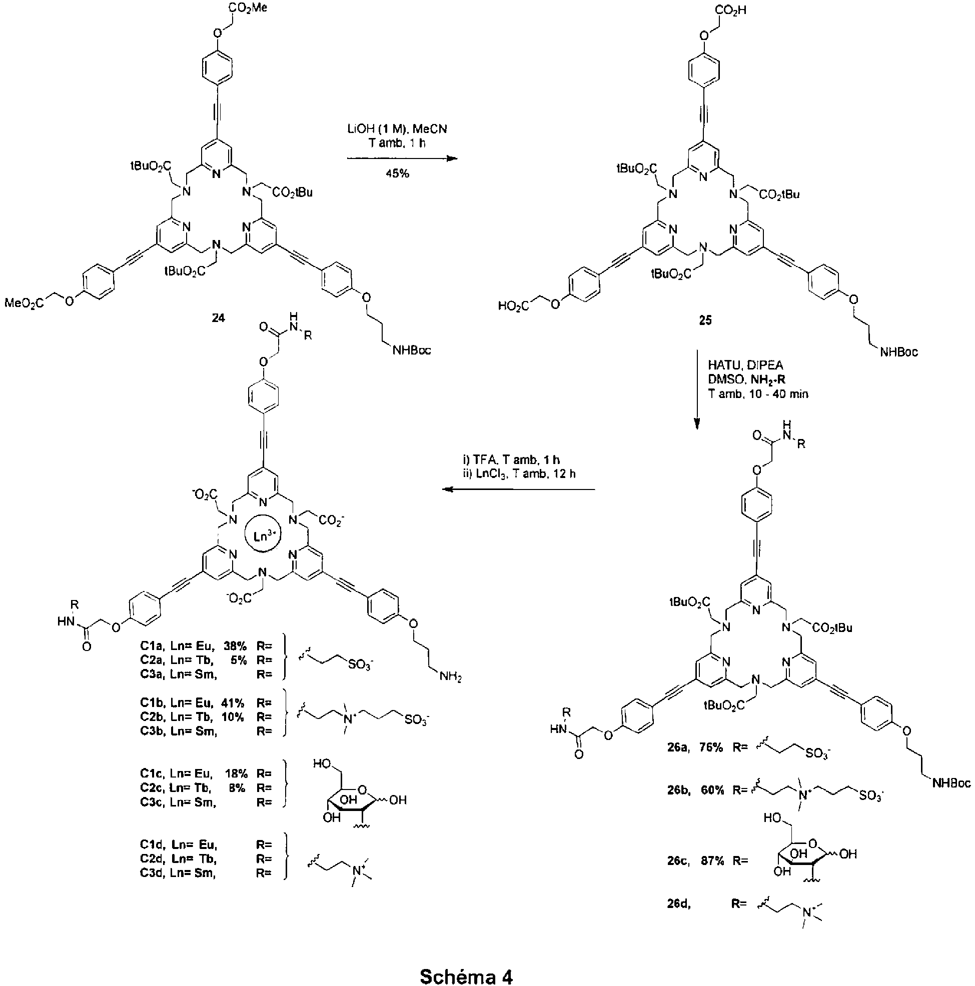

En utilisant une stratégie analogue les complexes de pyridinophane mono et di-antennes peuvent être préparés. Les schémas de synthèse sont décrits ci-après

### Synthèse systèmes mono antenne (pyridinyl-acétylène-4-O-phényl)

La synthèse de ces systèmes est décrite dans les schémas 5-7.

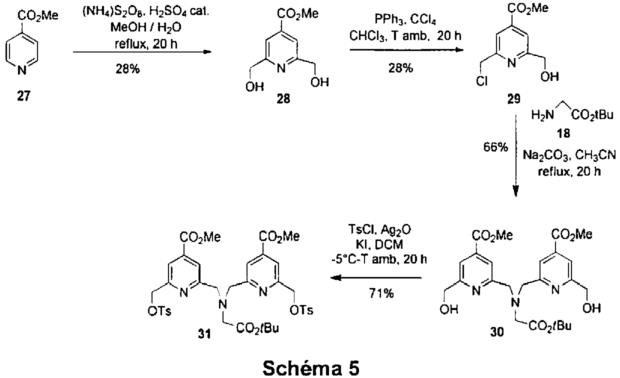

L'isonicotinate de méthyle traité dans des conditions oxydantes conduit au diol **28** avec un rendement de 28% ; Bien que ce rendement soit médiocre, cette réaction permet d'introduire simultanément deux groupements hydroxy-méthylène en position 2 et 6 du noyau pyridinyle et ceci en une seule étape. La mono-chloration du diol permet d'obtenir le composé **29** qui réagit ensuite avec le composé **18** conduisant au squelette di-pyridinyle **30.** Les fonctions alcools sont activées sous forme de tosylate puis l'étape de macrocyclisation est effectuée comme décrit précédemment sur les systèmes tri-antennes et conduit à l'intermédiaire clé **32.**

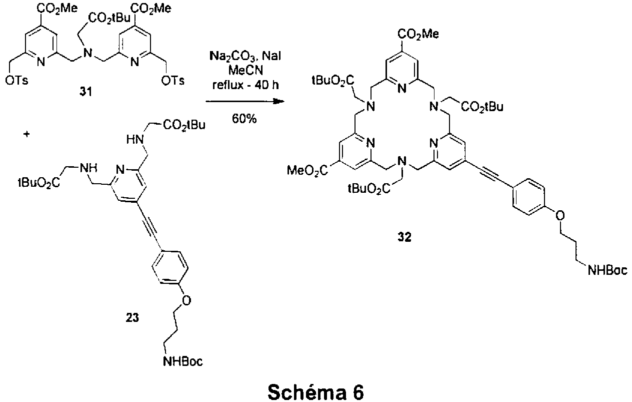

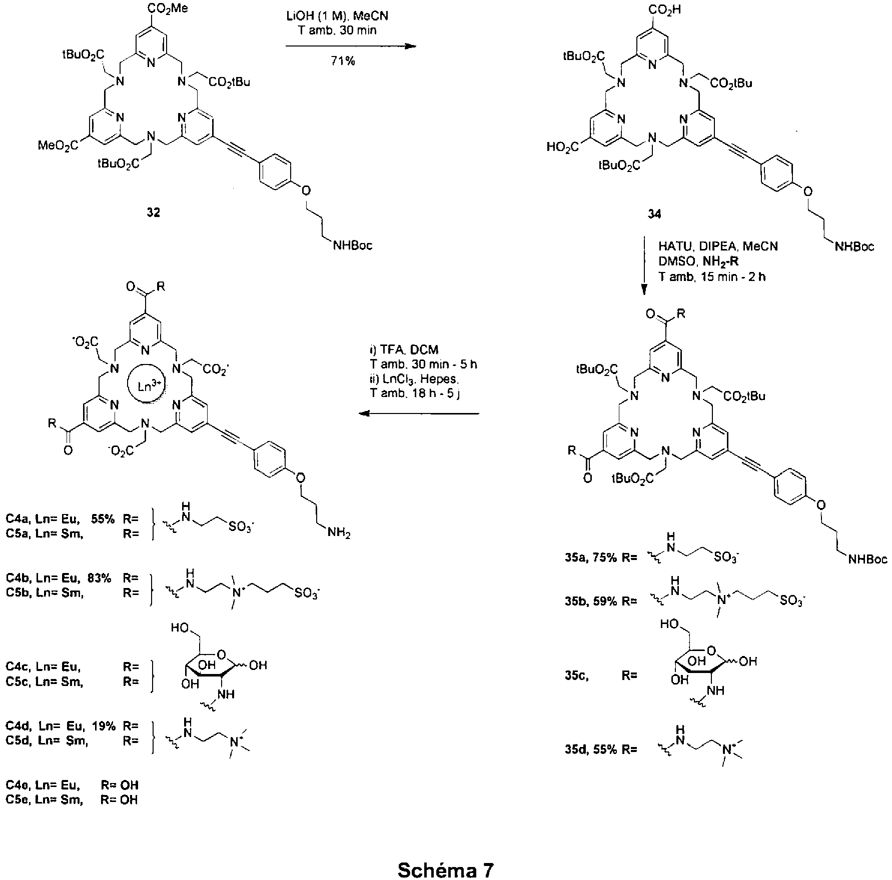

L'hydrolyse des deux fonctions ester méthylique conduit au composé di-acide **34** sur lequel sont greffées deux fonctions hydrosolubilisantes (de nature anionique **35a,** zwittérionique **35b,** neutre **35c,** ou cationique **35d**). La déprotection des esters butyliques et du groupement protecteur Boc est réalisée en présence d'acide trifluoroacétique pur. L'atome de lanthanide est complexé dans la cavité du ligand lorsque ce dernier est traité dans un tampon hepes en présence d'une solution aqueuse de chlorure d'europium ou de samarium.

### Synthèse des systèmes di-antennes (pyridinyl-acétylène-4-O-phényl)

La synthèse de ces systèmes est décrite dans les schémas 8-10.

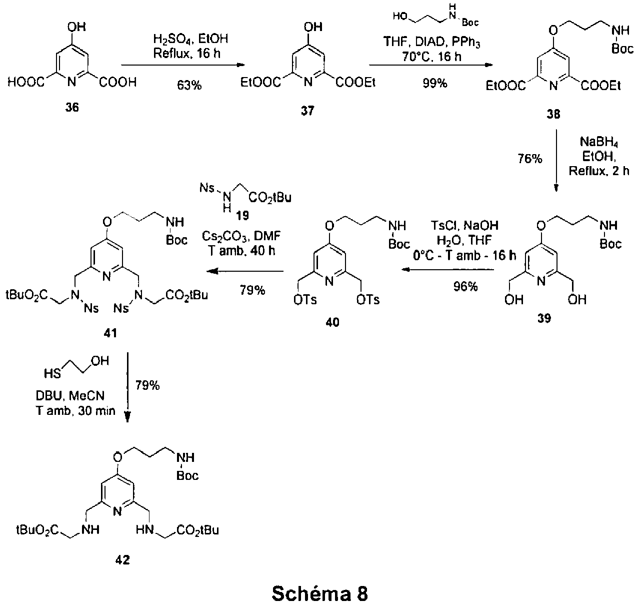

En ce qui concerne les systèmes di-antennes, la synthèse commence par une réaction d'estérification sur l'acide chélidamique **36** qui est disponible commercialement (procédure décrite dans Inorganic Chemistry 2000, 39, 4678). Le bras servant de lien entre le complexe et la biomolécule est introduit à ce niveau de la synthèse en utilisant une réaction de Mitsunobu (procédure décrite dans Organic Biomolecular Chemistry 2012, 10, 9183). Le diol 39 est obtenu par une double réaction de réduction en utilisant du borohydrure de sodium dans l'éthanol, puis ce dernier est ensuite activé sous forme ditosylée (procédure décrite dans Organic & Biomolecular Chemistry 2012, 10, 9183). Le synthon **42** est préparé comme précédemment (système tri-antennes) en condensant **19** avec **40** (préparé selon la procédure décrite dans Organic & Biomolecular Chemistry 2012, 10, 9183) puis en déprotégeant les groupements protecteurs nosyles. Les deux composés **12** et **42** sont utilisés pour la préparation de l'intermédiaire clé **43** constituant la base des systèmes di-antennes (schémas 9 et 10). 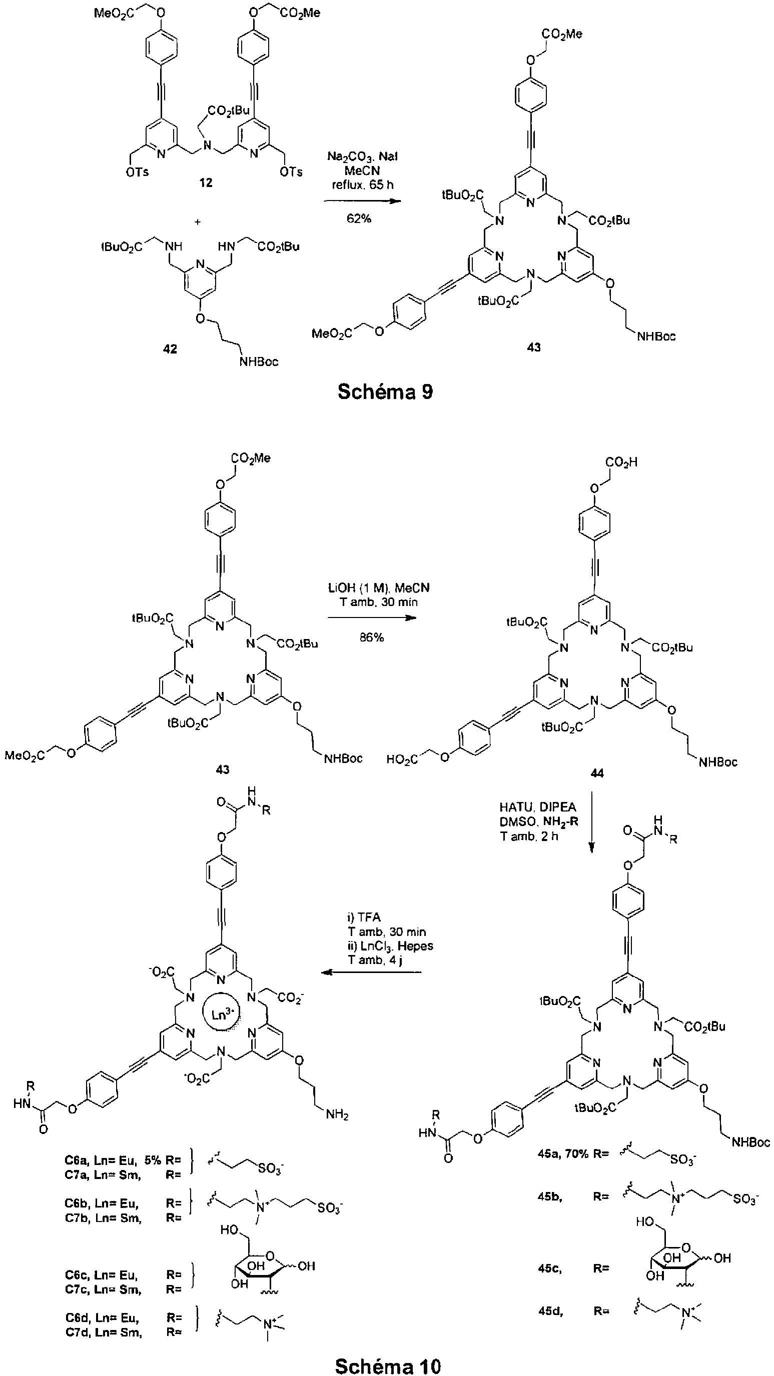 Les fonctions esters sont hydrolysées et les fonctions hydrosolubilisantes sont introduites comme précédemment mais cette fois sur les antennes directement. La suite de la synthèse est identique à ce qui a été décrit pour les tri et mono-antennes.

Ces sondes fluorescentes peuvent être activées en introduisant les fonctions classiques : ester de NHS, azido, maléimide ou isothiocyanate. Un exemple décrit l'activation sous forme de maléimide permettant la bioconjugaison sur des protéines, soit sur des messagers secondaires de type cAMP. Les schémas 11 et 12 décrivent deux exemples de ces molécules.

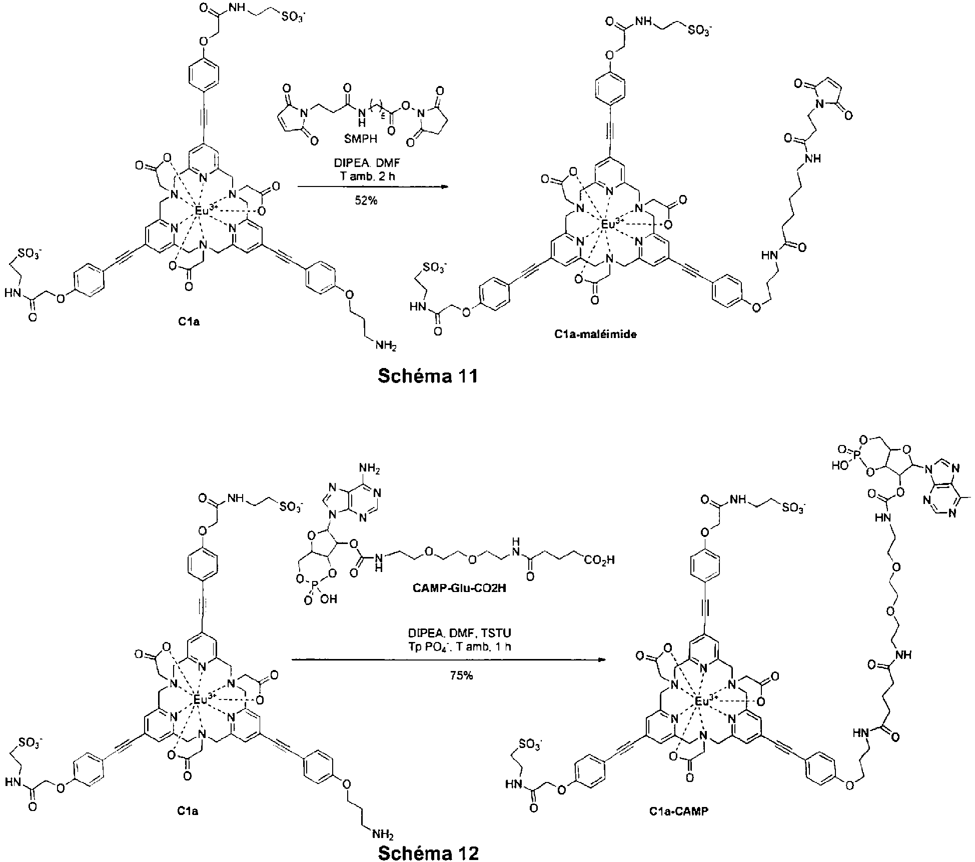

### Synthèse systèmes tri-antennes (pyridinyl-2,4,5-O-phényl)

Les systèmes pyridinyl triméthoxyphényl sont également des chromophores compatibles avec la technologie FRET. Les schémas 13-15 indiquent la méthodologie qui a été suivie pour l'obtention des complexes correspondants (série **C8-C10**). Les procédures sont identiques à celles décrites précédemment ou sont disponibles dans la littérature. Les détails sont donnés dans la partie expérimentale.

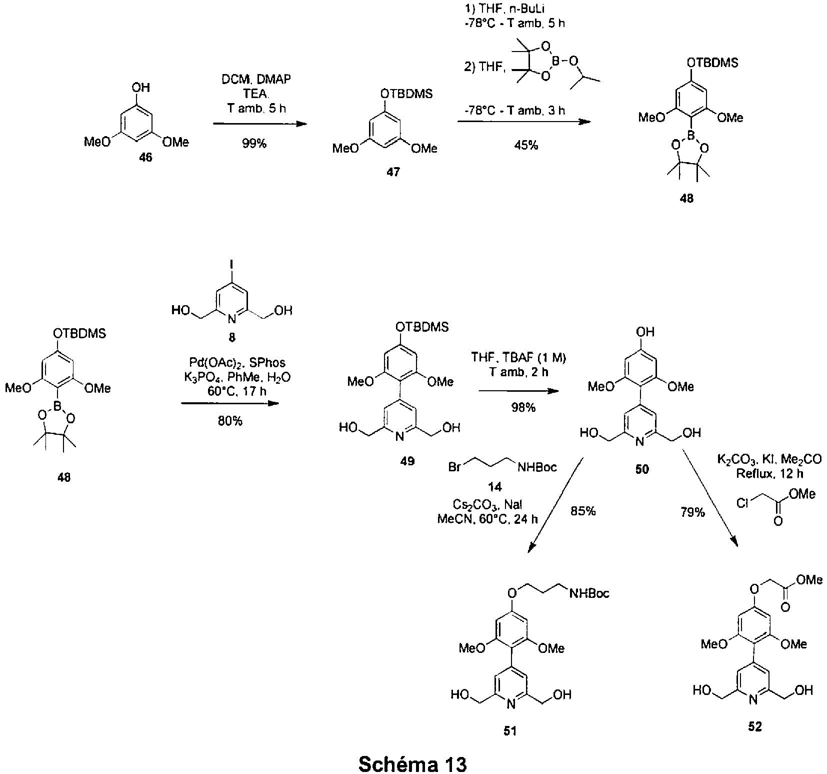

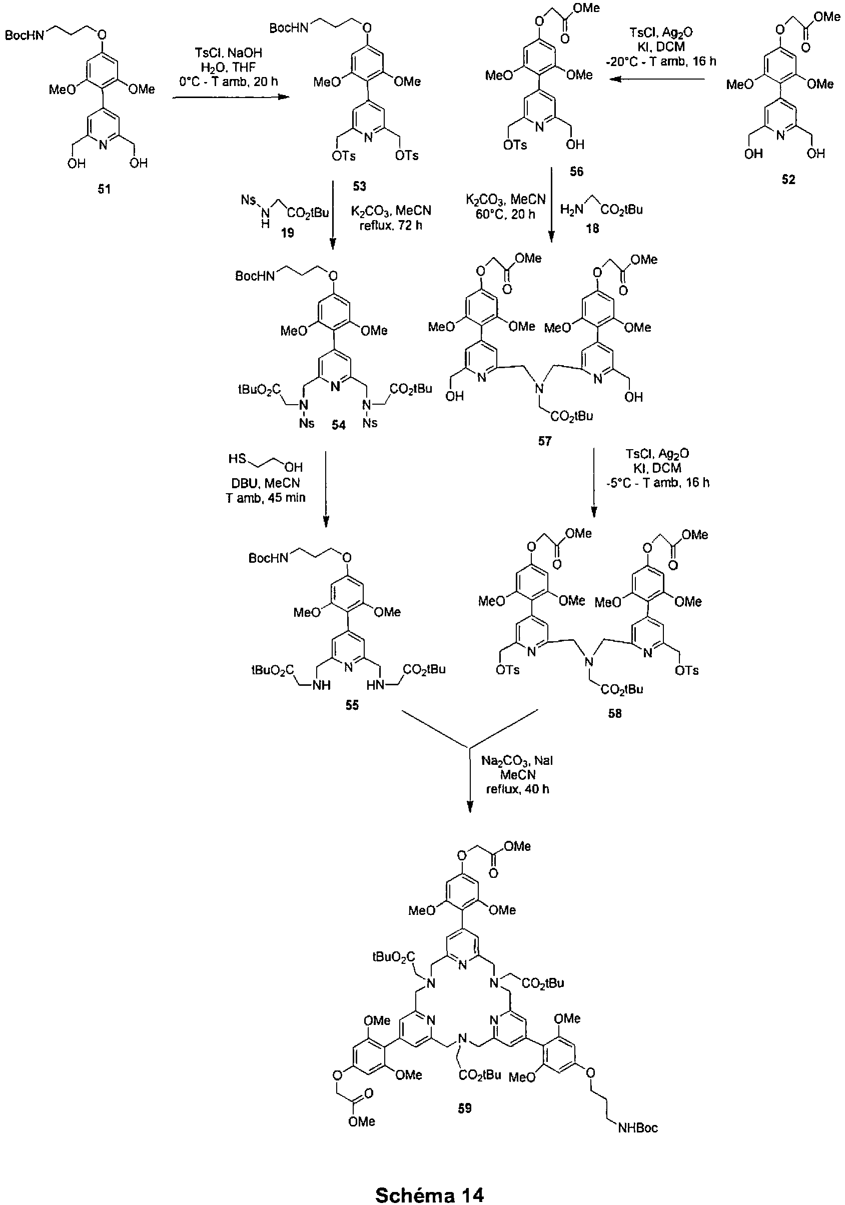

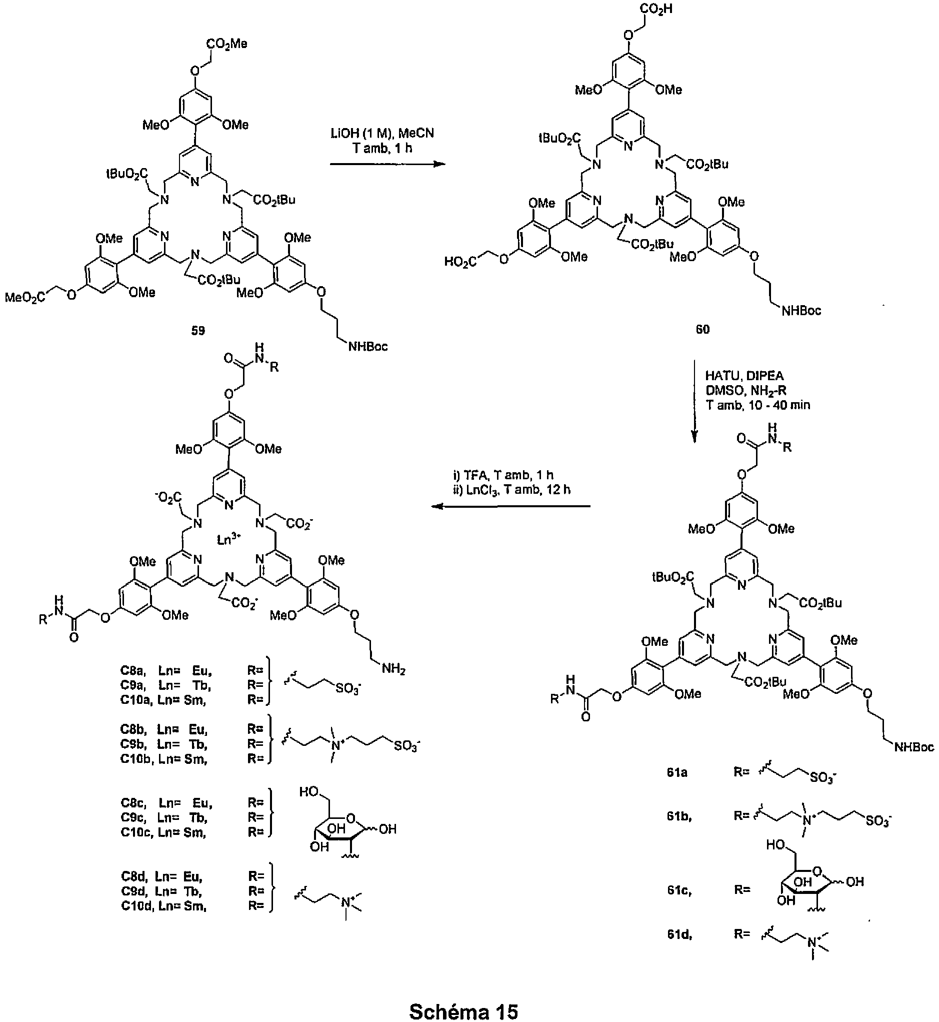

### Partie Expérimentale

### Informations générales

### Abréviations utilisées

Boc : *tert*-Butyloxycarbonyle
Boc-Osu : N-(*tert*-butoxycarbonyloxy)succinimide
cAMP : adénosylmonophosphate cyclique
CCM : chromatographie en couche mince
*m*-CPBA : acide métachloroperbenzoïque
DBU : 1,8-diazabicyclo[5.4.0]undéc-7-ène
DCC : dicyclohexylurée
DCM : dichlorométhane
δ: déplacement chimique
DIAD : diisopropyl azodicarboxylate
DIPEA : diisopropyléthylamine
DMF : diméthylformamide
DMSO : diméthyl sulfoxyde
EP : éther de pétrole
EtOH : éthanol
ESI : ionisation par électronébulisation
h : heure
HATU :1-[Bis(diméthylamino)méthylene]-1H-1,2,3-triazolo[4,5-b]pyridinium-3-oxyde hexafluorophosphate
HEPES : acide (4-(2-hydroxyéthyl)-1-pipérazine éthane sulfonique)
HPLC : chromatographie liquide à haute performance
HRMS : spectroscopie de masse à haute résolution
HTRF : Fluorescence Homogène en Temps Résolu
Hz : Hertz
j : jour
MeCN : acétonitrile
MeOH : méthanol
min : minute
Mops : acide 3-(N-morpholino)propanesulfonique
Ms : mésyle
NBS : N-bromosuccinimide
NHS : N-hydroxysuccinimide
NIS : N-iodosuccinimide
Ns : nosyle
PEG : polyéthylène glycol
Ph : phényle
ppm : partie par million
PtF : point de fusion
RMN : résonance magnétique nucléaire
SM : spectrométrie de masse
SMPH : hexanoate 6-((béta-maléimidopropionamido) de succinimidyle)
Sphos : 2-Dicyclohexylphosphino-2',6'-dimethoxybiphenyl
TA ou T amb : température ambiante
TEA ou Et₃N: triéthylamine
TFA :acide trifluoroacétique
THF : tétrahydrofurane
TMS : triméthylsilyle
TsCI : chlorure de tosyle
TSTU : tétrafluoroborate de O-(N-Succinimidyl)-1,1,3,3-tétraméthyluronium

### Chromatographie

Les chromatographies sur couches minces ont été effectuées sur des plaques de gel de silice Merck 60 F₂₅₄ sur feuille d'aluminium ou sur des plaques d'oxyde d'aluminium neutre Merck 60 F₂₅₄ (type E) sur feuille d'aluminium.

Les chromatographies liquides à haute performance (HPLC) analytiques et préparatives ont été effectuées sur deux appareils :
- HPLC Analytique : ThermoScientific, pompe quaternaire P4000, Détecteur UV 1000 à lampe au deutérium (190-350 nm), colonne analytique Waters XBridge C18, 3.5 µm, 4.6 × 100 mm.
- HPLC Préparative : Shimadzu, 2 pompes LC-8A, détecteur UV à barrette de diodes VarianProStar, colonne préparative Waters XBridgeprép. C18, 5 µm: 19 × 100 mm ou 50 × 150 mm.

Les chromatographies sur colonne de silice ont été réalisées sur gel de silice Merck 60 (0.040-0.063 mm). Les chromatographies sur colonne d'alumine ont été réalisées sur oxyde d'aluminium Sigma-Aldrich, neutre, activé, Brochmann I.

### Gradient A :

Colonne Waters Xbridge C₁₈, 300 Å, 3,5 µm, 4,6 x 100 mm, A/ eau 0,1 % acide formique - B/ acétonitrile 0,1 % acide formique, t = 0 min, 5 % B - t = 15 min 100 % B - 1 mL.min⁻¹.

### Gradient B :

Colonne Waters XBridge C₁₈, 5 µm, 19 x 100 mm, A / eau 0,1 % acide formique B / acétonitrile t = 0 min, 50 % B - t = 17 min, 100 % B - 20 mL.min⁻¹.

### Gradient C :

Colonne Waters XBridge C₁₈, 5 µm, 50 x 150 mm, A / eau 0,1 % acide formique B / acétonitrile t = 0 min, 15 % B - t = 2 min, 15 % B - t = 20 min, 100 % B - 100 mL.min⁻¹.

### Gradient D

Colonne Waters XBridge C₁₈, 5 µm, 19 x 100 mm, A / eau 0,1 % acide formique B / acétonitrile t = 0 min, 15 % B - t = 2min 15 % B - t = 20 min 100 % B - 20 mL.min⁻¹.

### Gradient E

Colonne Waters Xbridge C₁₈, 300 Å, 3,5 µm, 4,6 x 100 mm, A/ H₂O 5 mM acétate d'ammonium pH 6,5 - B/ acétonitrile - t = 0 min 2 % B - t = 15 min 40 % B. 1 mL.min⁻¹.

### Gradient F

Colonne Waters XBridge C₁₈, 5 µm, 50 x 150 mm, A / eau 25 mM acétate de triéthylammonium B / acétonitrile t = 0 min 2 % B - B - t = 17 min 40 % B - 100 mL.min⁻¹.

### Gradient G

Colonne Waters XBridge C₁₈, 5 µm, 19 x 100 mm, A / eau 25 mM acétate de triéthylammonium B / acétonitrile t = 0 min 5 % B - B - t = 17 min 40 % B - 20 mL.min⁻¹.

### Gradient H

Colonne Waters XBridge C₁₈, 300 Å, 5 µm, 10 x 100 mm - eau 25 mM acétate de triéthylammonium B / acétonitrile t = 0 min 2 % B - B - t = 19 min 40 % B - 5 mL.min⁻¹

### Gradient I

Colonne Waters Acquity C₁₈, 300 Å, 1,7 µm, 2,1 x 50 mm - A / eau 0,1% acide formique B / acétonitrile 0,1% acide formique t = 0 min 5 % B - t = 0,2 min 5 % B - t = 5 min 100 % B - 0,6 mL.min⁻¹

### Gradient J

Colonne Waters Xbridge C₁₈, 5 µm, 50 x 150 mm - A / eau 25 mM TEAAc pH 7 B / acétonitrile t = 0 min 50 % B - t = 20 min 100 % B - 100 mL.min⁻¹

### Gradient K

Colonne Waters Xbridge C₁₈, 5 µm, 50 x 150 mm - A / eau 25 mM TEAAc pH 7 B / acétonitrile t = 0 min 15 % B - t = 19 min 80 % B - 80 mL.min⁻¹

### Gradient L

Colonne Waters Xbridge C₁₈ 300 Å, 5 µm, 19 x 100 mm - A / eau 25 mM TEAAc pH 7 B / acétonitrile t = 0 min 20 % B - t = 19 min 80 % B - 100 mL.min⁻¹

### Gradient M

Colonne Waters Xbridge C₁₈, 5 µm, 50 x 150 mm - A / eau 25 mM TEAAc pH 7 B / acétonitrile t = 0 min 2 % B - t = 18 min 40 % B - 80 mL.min⁻¹

### Gradient N

Colonne Waters Acquity C₁₈, 300 Å, 1,7 µm, 2,1 x 50 mm - A / eau 5 mM acétate d'ammonium pH 5 B / acétonitrile t = 0 min 2 % B - t = 0,2 min 2 % B - t = 5 min 40 % B - 0,6 mL.min⁻¹

### Gradient O

Colonne Waters Xbridge C₁₈, 5 µm, 50 x 150 mm - A / eau 0,1 % acide formique B / acétonitrile t = 0 min 40 % B - t = 20 min 100 % B - 100 mL.min⁻¹

### Spectroscopie

### a. Résonance magnétique nucléaire

Les spectres RMN (¹H, ¹³C et ³¹P) ont été réalisés à l'aide d'un spectromètre Bruker Avance 400 MHz NanoBay (aimant de 9,4 Teslas), muni d'une sonde de mesure BBFO, multi noyaux de diamètre de 5 mm, de gradient Z et de lock²H. Les déplacements chimiques (δ) sont exprimés en partie par million (ppm). Les abréviations suivantes sont utilisées :
s : singulet, se : singulet élargi, d : doublet, t : triplet, q : quadruplet, m : multiplet, dd : doublet dédoublé, td : triplet dédoublé, qd : quadruplet dédoublé, ddd : doublet de doublet dédoublé.

### b. Spectrométrie de masse

Les spectres de masse (LC-MS) ont été réalisés à l'aide d'un spectromètre Waters ZQ 2000 simple quadipôle à source multimode ESI/APCI équipé de colonne Waters XBridge C₁₈, 3.5µm, 4.6 × 100 mm.

### c. Spectrométrie de masse haute résolution

Les analyses ont été effectuées avec un spectromètre de masse QStar Elite (AppliedBiosystems SCIEX) équipé d'une source d'ionisation à pression atmosphérique (API) assistée pneumatiquement. L'échantillon a été ionisé en mode electrospray positif dans les conditions suivantes : tension electrospray (ISV) : 5500 V ; tension d'orifice (OR) : 20 V ; pression du gaz de nébulisation (air) : 20 psi. Le spectre de masse haute résolution (MS) a été obtenu avec un analyseur temps de vol (TOF). La mesure de masse exacte a été effectuée en triplicat avec un double étalonnage interne.

### Divers

Appareil à point de fusion : les points de fusion ont été réalisés en utilisant un appareil BUCHI melting point B-540.
**Composé 1** : disponible commercialement.
**Composé 2** : le composé **2** a été préparé selon la procédure décrite dans WO 2004/094386.
**Composé 3 :** le composé 3 a été préparé selon la procédure décrite dans l'article : Journal of Medicinal Chemistry 2013, 56,4990.
**Composé 4**
   A une solution du composé **3** (20 g, 38,1 mmol) dans du DCM anhydre (20 mL) ont été ajoutés successivement à 0°C du MeOH anhydre (30 mL) et du carbonate de potassium anhydre (15,8 g, 114 mmol). La suspension a été agitée à 0°C pendant 15 min puis à TA pendant 1 h puis a été filtrée et concentrée sous pression réduite. Le résidu a été dilué avec du DCM (100 mL) et ensuite a été ajouté de l'eau (80 mL). La phase organique a été séparée et la phase aqueuse a été extraite avec du DCM (2 x 60 mL). Les phases organiques ont été réunies, séchées sur sulfate de sodium, filtrées et concentrées sous pression réduite pour conduire au composé **4** (12 g, 83%) comme un solide légèrement marron qui a été utilisé dans la suite de la synthèse sans purification supplémentaire. RMN ¹H (CDCl₃, 400 MHz) δ 7.45 (d, *J* = 8Hz, 2H), 6.86 (d, *J* = 8 Hz, 2H), 4.65 (s, 2H), 3.82 (s, 3H), 3.03 (s, 1H); RMN ¹³C (CDCl₃, 100 MHz) δ 168.9, 158.0, 133.6, 115.4, 114.6, 83.3, 76.3, 65.1, 52.3. LRMS (ESI+) : calculée pour C₁₁H₁₁O₃ [M+H]⁺, *m*/*z* 191.07, trouvée 191.70.
**Composé 5** : disponible commercialement.
**Composé 6 :** le composé **6** a été préparé selon la procédure décrite dans l'article : Tetrahedron 2005, 61, 1755.
**Composé 7** : le composé **7** a été préparé selon la procédure décrite dans l'article : Tetrahedron 2008, 64, 399.
**Composé 8** : le composé **8** a été préparé selon la procédure décrite dans l'article : European Journal of Organic Chemistry 2002, 21, 3680.
**Composé 9**
   A une suspension du composé **8** (3 g, 11.3 mmol) dans du DCM anhydre (50 mL) refroidie à -20 °C ont été ajoutés de l'oxyde d'argent (3,93 g, 17,0 mmol), de l'iodure de potassium (0,37 g, 2,2 mmol) puis par petites portions du TsCI (2,37 g, 12,5 mmol). Le mélange a été agité à TA pendant 16 h puis filtré sur Celite. Le filtrat a été concentré sous pression réduite et l'huile résiduelle a été purifiée par chromatographie sur colonne de silice en utilisant du DCM comme éluant pour conduire à un solide blanc correspondant au composé 9 (2,1 g, 44%). Pt de fusion : 97-98°C. RMN ¹H (CDCl₃, 400 MHz) δ 7.84 (d, *J* = 8.4 Hz, 2H), 7.64 (s, 1H), 7.62 (s, 1H), 7.38 (d, *J* = 8.4 Hz, 2H), 5.10 (s, 2H), 4.67 (s, 2H), 3.20 (bs, 1H), 2.48 (s, 3H); RMN ¹³C (CDCl₃, 100 MHz) δ 160.0, 153.4, 145.4, 132.6, 130.0, 129.4, 129.3, 128.0, 106.9, 70.5, 63.4, 21.7. HRMS (ESI+) : calculée pour C₁₄H₁₅NO₄IS [M+H]⁺, *m*/*z* 419.9766, trouvée 419.9759.
**Composé 10**
   A une suspension d'hydrochlorure de glycinate de *tert*-butyle (0,76 g, 4,5 mmol) dans MeCN anhydre (76 mL) a été ajouté du carbonate de potassium anhydre (1,88 g, 13,6 mmol) et ce mélange a été chauffé à 55°C pendant 30 min puis refroidi à TA. A cette suspension a été ajouté le composé 9 (4,0 g, 9 mmol) puis le mélange a été chauffé à 60°C pendant 20 h. Lorsque le mélange est revenu à TA, le mélange a été filtré et le filtrat a été concentré sous pression réduite et le résidu a été purifié par chromatographie sur colonne de silice en utilisant un gradient de solvant AcOEt - MeOH 97/3 jusqu'à 90/10 par incrément de 1% pour conduire au composé 10 (2,5 g, 90%) sous forme de mousse. RMN ¹H (CDCl₃, 400 MHz) δ 7.79 (s, 2H), 7.53 (s, 2H), 4.70 (s, 4H), 3.96 (s, 4H), 3.86 (bs, 2H), 3.40 (s, 2H), 1.53 (s, 9H); RMN ¹³C (CDCl₃, 100 MHz) δ 170.1, 159.6, 158.8, 131.0, 128.3, 106.8, 81.6, 63.6, 59.2, 56.7, 38.2. HRMS (ESI+) : calculée pour C₂₀H₂₆N₃O₄I [M+H]⁺, *m*/*z* 626.0013, trouvée 626.0015.
**Composé 11**
   Une solution de composé 10 (1,64 g, 2,62 mmol) et de composé **4** (1,5 g, 7,87 mmol) dans un mélange THF (7,5 mL) et TEA (7,5 mL) a été dégazée par un courant d'argon pendant 15 min. Puis à ce mélange ont été ajoutés du 1,1'-bis(diphénylphosphino)ferrocène]dichloropalladium(II) (0,64 g, 0,78 mmol) et de l'iodure de cuivre (0,3 g, 1,57 mmol). Ce mélange a été irradié sous microondes (100 W) pendant 30 min puis refroidi à TA et enfin filtré sur un lit de Celite. Le filtrat a été concentré sous pression réduite et le résidu a été purifié par chromatographie sur colonne de silice en utilisant un gradient de solvant DCM/AcOEt/MeOH de 1/0/0, 5/5/0, 0/1/0 jusqu'à 0/9/1 par incrément de 5% pour conduire au composé 11 souhaité (1,39 g, 71%). RMN ¹H (CDCl₃, 400 MHz) δ 7.51 (s, 2H), 7.48 (d, *J* = 8.7 Hz, 4H), 7.19 (s, 2H), 6.87 (d, *J* = 8.7 Hz, 4H), 4.75 (s, 4H), 4.68 (s, 4H), 4.16 (bs, 2H), 4.03 (s, 4H), 3.84 (s, 6H), 3.43 (s, 2H), 1.53 (s, 9H); RMN ¹³C (CDCl₃, 100 MHz) δ 170.4, 168.9, 158.5, 158.3, 158.0, 133.6, 132.6, 123.7, 120.5, 115.3, 114.7, 93.6, 86.2, 81.4, 65.1, 63.9, 59.5, 56.4, 52.3, 28.2. HRMS (ESI+) : calculée pour C₄₂H₄₄N₃O₁₀ [M+H]⁺, *m*/*z* 750.3027, trouvée 750.3022.
**Composé 12**
   A une solution de diol **11** (250 mg, 0,33 mmol) dans du DCM (8 mL) ont été ajoutés de l'iodure de potassium (22 mg, 0,14 mmol) et de l'oxyde d'argent (231 mg, 1 mmol). A ce mélange refroidi à - 5°C a été ajouté en une fois du TsCI (190 mg, 1 mmol). Le mélange a été ensuite agité à TA pendant 16 h. L'avancement de la réaction a été suivi par CCM. Après cette période, le solvant a été éliminé sous pression et le résidu a été purifié par chromatographie sur colonne de silice en utilisant un gradient d'éluant DCM-MeOH de 99/1 jusqu'à 98/2 par incrément de 0,2 % pour conduire à une huile beige (300 mg, 88%). RMN ¹H (CDCl₃; 300 MHz) δ 1.46 (s, 9H); 2.4 (s, 6H), 3.32 (s, 2H), 3.80 (s, 6H), 3.87 (s, 4H), 4.65 (s, 4H), 5.07 (s, 4H), 6.83 (d, *J* = 8.9 Hz, 4H), 7.28 (s, 2H), 7.29 (d, *J* = 8.3 Hz, 4H), 7.44 (d, *J* = 8.9 Hz, 4H), 7.52 (s, 2H), 7.79 (d, *J* = 8.3 Hz, 4H). RMN ¹³C (CDCl₃ ; 75 MHz) δ 170.0, 168.8. 159.0, 158.3, 153.1, 145.0, 133.5, 133.0, 132.6, 129.8, 128.0, 124.2, 121.6, 115.1, 114.7, 94.1, 85.9, 81.2, 71.3, 65.0, 59.5, 56.0, 52.3, 28.1, 21.5. HRMS (ESI+) : calculée pour C₅₆H₅₆N₃O₁₄S₂ [M+H]⁺, *m*/*z* 1058.3204, trouvée 1058.3241.
**Composé 13** : disponible commercialement.
**Composé 14:** disponible commercialement.
**Composé 15:** le composé **15** a été préparé selon la procédure décrite dans l'article: Organic Letters 2006, 8, 4251.
**Composé 16 :** le composé **16** a été préparé selon la procédure décrite dans l'article : Organic Letters. 2006, 8, 4251.
**Composé 17** : le composé **17** a été préparé selon la procédure décrite dans la demande WO 2014/111661.
**Composé 18** : disponible commercialement.
**Composé 19** : le composé **19** a été préparé selon la procédure décrite dans l'article : Organic Letters 2007, 9, 1635.
**Composé 20**
   A une solution de composé **8** (3,0 g, 11,3 mmol) dans du THF (10 mL) a été ajoutée une solution aqueuse de soude (1,36 g, 34 mmol dissous dans 10 mL d'eau) à TA. A ce mélange refroidi à 0°C a été additionné une solution de TsCI (6,47 g, 34 mmol) dans du THF (10 mL) et a été agité pendant 20 h à TA. A cette suspension ont été ajoutés du DCM (100 mL) et une solution saturée de saumure (50 mL). Après décantation, la phase aqueuse a été extraite par du DCM (2 x 100 mL). Les phases organiques ont été réunies, séchées sur sulfate de sodium, filtrées et concentrées sous pression réduite. Le produit brut a été purifié par chromatographie sur colonne de silice avec un gradient d'éluant cyclohexane/DCM de 50/50 jusqu'à 0/100 pour conduire au composé 20 (5,41 g, 84%) comme un solide blanc. Pt de fusion : 143.5-144.5°C. RMN ¹H (CDCl₃, 400 MHz) δ 7.81 (d, *J* = 8.2 Hz, 4H), 7.64 (s, 2H), 7.36 (d, *J* = 8.2 Hz, 4H), 5.01 (s, 4H), 2.47 (s, 6H); RMN ¹³C (CDCl₃, 100 MHz) δ 154.1, 145.3, 132.6, 130.3, 130.0, 128.0, 106.9, 70.38, 21.72. HRMS (ESI+) : calculée pour C₂₁H₂₁NO₆IS₂ [M+H]⁺, *m*/*z* 573.9855, trouvée 573.9854.
**Composé 21**
   A une solution de composé **19** (1,83 g, 5,75 mmol) dans MeCN anhydre (36 mL) a été additionné du carbonate de sodium (2,77 g, 26,2 mmol) puis la suspension a été agitée à 80°C pendant 1 h. A cette suspension refroidie à TA a été ajouté le composé **20** (1,5 g, 2,62 mmol) et le mélange a été agité à 80°C pendant 72 h. Après cette période, le mélange réactionnel a été refroidi à TA, filtré et le filtrat a été concentré sous pression réduite. Le résidu été purifié par chromatographie sur colonne de silice en utilisant un gradient d'éluant AcOEt/MeOH de 98/2 jusqu'à 95/5 par incrément de 0,5% pour conduire au composé **21** (2,0 g, 89%) comme une huile jaunâtre. RMN ¹H (CDCl₃, 400 MHz) δ 8.08 (dd, *J* = 7.6 Hz, 1.6 Hz, 2H), 7.75-7.66 (m, 6H), 7.60 (s, 2H), 4.63 (s, 4H), 4.15 (s, 4H), 1.39 (s, 18H); RMN ¹³C (CDCl₃, 100 MHz) δ 167.3, 156.3, 147.7, 133.7, 133.5, 131.7, 130.8, 130.4, 124.2, 107.3, 82.6, 52.8, 49.2, 28.0. HRMS (ESI+) : calculée pour C₃₁H₃₇N₅O₁₂IS₂ [M+H]⁺, *m*/*z* 862.0925, trouvée 862.0927.
**Composé 22**
   A une solution de composé **17** (2,58 g, 9,4 mmol) et de composé **21** (8,2 g, 8,54 mmol) dans un mélange de TEA (42 mL) et de THF anhydre (42 mL) a été dégazée sous un courant d'argon pendant 15 min. Puis à ce mélange ont été ajouté du 1,1'-bis(diphénylphosphino)ferrocène] dichloropalladium(II) (1,39 g, 1,70 mmol) et de l'iodure de cuivre (0,49 g, 1,57 mmol) et a été chauffé à 60°C sous agitation pendant 20 h. Ce mélange a été refroidi à TA et filtré sur un lit de Celite. Le filtrat a été concentré sous pression réduite et le produit brut a été purifié par chromatographie sur colonne de silice en utilisant un gradient d'éluant cyclohexane/DCM/AcOEt de 5/2/3 jusqu'à 2/5/3 pour conduire au composé **22** (4,9 g, 57%) comme une mousse jaune. RMN ¹H (CDCl₃, 400 MHz) δ 8.10 (dd, *J* = 7.6 Hz, 1.7 Hz, 2H), 7.75-7-65 (m, 6H), 7.49 (d, *J* = 8.7 Hz, 2H), 7.32 (s, 2H), 6.92 (d, *J* = 8.7 Hz, 2H), 4.76 (bs, 1H), 4.68 (s, 4H), 4.17 (s, 4H), 4.08 (t, *J* = 6.0Hz, 2H), 3.36 (m, 2H), 2.03 (m, 2H), 1.47 (s, 9H), 1.38 (s, 18H); RMN ¹³C (CDCl₃, 100 MHz) δ 167.4, 159.7, 156.0, 155.8, 147.9, 133.8, 133.6, 133.6, 131.8, 130.8, 124.2, 122.7, 114.7, 113.8, 95.3, 85.5, 82.4, 79.3, 65.9, 53.0, 49.2, 37.8, 29.5, 28.4, 27.9. HRMS (ESI+) : calculée pour C₄₇H₅₇N₆O₁₅S₂ [M+H]⁺, *m*/*z* 1009.3323 trouvée 1009.3322.
**Composé 23**
   A une solution de composé **22** (0,1 g, 0,099 mmol) dans MeCN (4,3 mL), ont été additionnés sous agitation du 2-mercaptoéthanol (76 µL, 1,09 mmol) et du 1, 8 diazabicyclo [5-4-0] unde-7-ène (67 µL, 0,445 mol). La réaction a été agitée pendant 45 min à TA. L'avancement de la réaction a été suivi par CCM. Après cette période la réaction était totale. Le solvant a été éliminé sous pression réduite et le produit brut a été purifié par chromatographie sur colonne de silice en utilisant comme éluant un mélange AcOEt/MeOH (98/02 puis 96/04) pour conduire au composé **23** (35 mg, 55%) comme une huile beige. RMN ¹H (CDCl₃, 300 MHz) δ 1.46 (s, 9H), 1.49 (s, 18H), 2.01 (m, 2H), 3.33 (m, 2H), 3.40 (s, 4H), 3.94 (s, 4H), 4.06 (m, 2H), 6.89 (d, *J* = 8.8 Hz, 2H); 7.32 (s, 2H); 7.48 (d, J = 8.8 Hz, 2H). RMN ¹³C (CDCl₃, 75 MHz) δ, 171.3, 159.4,159.0, 156.0, 133.4, 132.6, 122.0, 114.6, 114.3, 93.7, 86.1, 81.2, 79.2, 65.8, 54.3, 51.1, 37.8, 29.5, 28.3, 28.1, HRMS (ESI+) : calculée pour C₃₅H₅₁N₄O₇ [M+H]⁺, *m*/*z* 639.3758, trouvée 639.3784.
**Composé 24**
   A une solution de dérivé ditosylé **12** (312 mg, 0,295 mmol) et de dérivé **23** (188 mg, 0,295 mmol) dans MeCN anhydre (140 mL) ont été additionnés du carbonate de sodium (312 mg, 2,95 mmol) et de l'iodure de sodium (4.4 mg, 0,0295 mmol). La suspension a été agitée à reflux pendant 40 h. L'avancement de la réaction a été suivi par CCM. Après cette période la réaction était totale. Le mélange réactionnel a été refroidi à TA, filtré et le filtrat a été concentré sous pression réduite. Le résidu a été purifié par chromatographie sur colonne d'alumine en utilisant comme éluant un mélange DCM/MeOH (98/02 puis 97/03) pour conduire au composé **24** (297 mg, 74%) comme une huile beige. RMN ¹H (CDCl₃, 300 MHz) δ 1.47(s, 9H), 1.52 (s, 27H), 2.02 (m, 2H), 3.37 (m, 2H), 3.43 (s, 4H), 3.45 (s, 2H), 3.84 (s, 6H), 3.90 (m, 12H), 4.02 (m, 2H), 4.62 (s, 4H), 6.69 (m, 6H), 7.22 (m, 4H), 7.25 (s, 2H), 7.35 (m, 6H). RMN ¹³C (CDCl₃, 75 MHz) δ 170.6, 168.9, 159.1, 158.49, 158.45, 158.4, 157.8, 156.0, 133.43, 133.41, 132.0, 131.7, 123.0, 122.97, 122.8, 115.8, 114.6, 114.4, 114.3, 92.9, 92.4, 86.6, 86.4, 81.1, 79.2, 65.7, 65.0, 60.3, 60.2, 60.1, 59.0, 58.7, 52.3, 37.7, 29.4, 28.4, 28.2. HRMS (ESI+): calculée pour C₇₇H₉₀N₇O₁₅ [M+H]⁺, *m*/*z* 1352.6495, trouvée 1352.6521.
**Composé 25**
   A une solution de composé **24** (21,1 mg, 15,6 µmol) dans MeCN (2,11 mL) a été ajouté une solution aqueuse d'hydroxyde de lithium 1 M (156 µL, 156 µmol). La solution a été agitée à TA pendant 1 h. L'avancement de la réaction a été suivi par LC-MS (gradient A). Après cette période, la réaction était totale. La solution a été directement purifiée par HPLC préparative (gradient B) et a conduit au composé **25** (9,4 mg, 45%) identifié comme le composé souhaité. HRMS (ESI+) : calculée pour C₇₅H₈₆N₇O₁₅ [M+H]⁺, *m*/*z* 1324.6182, trouvée 1324.6185. HPLC (gradient A) *Tr =* 13.95 min.
**Composé 26a**
   A une solution du composé **25** (34 mg, 25,7 µmol) dans du DMSO anhydre (1275 µL) ont été ajouté de la taurine (12,85 mg, 102,7 µmol), de la DIPEA (26,4 µL, 19,9 mg, 154 µmol) et enfin du HATU (39 mg, 102,7 µmol). La solution a été agitée à TA pendant 40 min. L'avancement de la réaction a été suivi par LC-MS (gradient A). Après cette période, la réaction était totale. La solution a été directement purifiée par HPLC préparative (gradient C) et a conduit au composé 26a (30 mg, 76%) identifié comme le composé souhaité. HRMS (ESI+) : calculée pour C₇₉H₉₇N₉O₁₉S₂ [M+2H]²⁺, *m*/*z* 769.8171, trouvée 769.8166. HPLC (gradient A) *Tr* = 11.33 min.
**Composé 26b**
   Le 3-((2-aminoéthyl)diméthylammonio)propane-1-sulfonate a été préparé selon la procédure décrite dans WO 2011/146595 et dans Organic & Biomolecular Chemistry 2012, 10, 1883.
   A une solution du composé **25** (15 mg, 11,3 µmol) dans du DMSO anhydre (562 µL) ont été ajouté du 3-((2-aminoéthyl)diméthylammonio)propane-1-sulfonate (15 mg, 71,58 µmol), de la DIPEA (11,6 µL, 8,8 mg, 67,9 µmol) et enfin du HATU (17,2 mg, 45,3 µmol). La solution a été agitée à TA pendant 10 min. L'avancement de la réaction a été suivi par LC-MS (gradient A). Après cette période, la réaction était totale. La solution a été directement purifiée par HPLC préparative (gradient D) et a conduit au composé **26b** (11.6 mg, 60%) identifié comme le composé souhaité. HRMS (ESI+) : calculée pour C₈₉H₁₁₉N₁₁O₁₉S₂ [M+2H]²⁺, *m*/*z* 854.9062, trouvée 854.9054. HPLC (gradient A) *Tr* = 11.14 min.
**Composé 26c**
   A une solution du composé **25** (15 mg, 11,3 µmol) dans du DMSO anhydre (562 µL) ont été ajouté de la glucosamine (9,77 mg, 45,3 µmol), de la DIPEA (11,6 µL, 8,8 mg, 67,9 µmol) et enfin du HATU (17,2 mg, 45,3 µmol). La solution a été agitée à TA pendant 10 min. L'avancement de la réaction a été suivi par LC-MS (gradient A). Après cette période, la réaction était totale. La solution a été directement purifiée par HPLC préparative (gradient D) et a conduit au composé 26c (16,3 mg, 87%) identifié comme le composé souhaité. HRMS (ESI+) : calculée pour C₈₇H₁₀₈N₉O₂₃ [M+H]⁺, *m*/*z* 1646.7558, trouvée 1646.7558. HPLC (gradient A) *Tr* = 11.62 min.
**Composé 26d** : ce composé a été préparé selon la même procédure que celle utilisée pour la synthèse de **26a-c** en sélectionnant l'amine correspondante.
**Complexes C1a** - **C2a-C3a**
   Au composé **26a** (30 mg, 19,5 µmol) a été ajouté du TFA (500 µL). La solution a été agitée à TA pendant 2 h. L'avancement de la réaction a été suivi par LC-MS (gradient A). Après cette période, la réaction était totale. Le solvant a été éliminé sous pression réduite. Le résidu a été divisé en deux parties. Une partie a été utilisée pour la préparation du complexe d'europium **C1a** et l'autre partie pour la préparation du complexe de terbium **C2a.**
**C1a**
   Au résidu (22,2 mg, 18 µmol) ont été ajouté de l'eau (6 mL), MeCN (2 mL) et une solution aqueuse d'hydroxyde de soude 3 M afin d'obtenir une solution pH 7. A cette solution ont été ajouté du chlorure d'europium (26,4 mg, 72 µmol) et une solution aqueuse d'hydroxyde de soude 3 M afin d'obtenir une solution pH 6. La solution a été agitée à TA pendant une nuit. L'avancement de la réaction a été suivi par LC-MS (gradient E). Après cette période, la réaction était totale. La solution a été directement purifiée par HPLC préparative (gradient F) et a conduit au complexe **C1a** (9,7 mg, 38%) identifié comme le composé souhaité. HRMS (ESI+) : calculée pour C₆₂H₆₃N₉O₁₇S₂Eu [M]³⁺, *m*/*z* 473.4322, trouvée 473.4319. HPLC (gradient E).
**C2a**
   Au résidu obtenu lors de la première étape (1,9 mg, 1,5 µmol) ont été ajoutés de l'eau (375 µL), du chlorure de terbium (2,24 mg, 6 µmol) et une solution aqueuse d'hydroxyde de soude 3 M afin d'obtenir une solution pH 6. La solution a été agitée à TA pendant une nuit. L'avancement de la réaction a été suivi par LC-MS (gradient E). Après cette période, la réaction était totale. La solution a été directement purifiée par HPLC préparative (gradient G) et a conduit au complexe **C2a** (107 µg, 5%) identifié comme le composé souhaité. HRMS (ESI+) : calculée pour C₆₂H₆₃N₉O₁₇S₂Tb [M+3H]³⁺, *m*/*z* 476.1012, trouvée 476.1005. HPLC (gradient E).
**C3a** : ce complexe a été préparé selon la même procédure que celle utilisée pour la synthèse de **C1a** et **C2a.**
**Complexes C1b** - **C2b** - **C3b**
   Au composé **26b** (11,6 mg, 7 µmol) a été ajouté du TFA (250 µL). La solution a été agitée à TA pendant 2 h. L'avancement de la réaction a été suivi par LC-MS (gradient A). Après cette période, la réaction était totale. Le solvant a été éliminé sous pression réduite. Le résidu a été divisé en deux parties. Une partie a été utilisée pour la préparation du complexe d'europium **C1b** et l'autre partie pour la préparation du complexe de terbium **C2b.**
**C1b**
   Au résidu obtenu précédemment (7,9 mg, 5,5 µmol) ont été ajoutés de l'eau (3,15 mL), du chlorure d'europium (8,1 mg, 22 µmol) et une solution aqueuse d'hydroxyde de soude 3 M afin d'obtenir une solution pH 6. La solution a été agitée à TA pendant une nuit. L'avancement de la réaction a été suivi par LC-MS (gradient E). Après cette période, la réaction était totale. La solution a été directement purifiée par HPLC préparative (gradient F) et a conduit au complexe **C1b** (3.58 mg, 41%) identifié comme le composé souhaité. HRMS (ESI+) : calculée pour C₇₂H₈₃N₁₁O₁₇S₂Eu [M+H]³⁺, *m*/*z* 530.1540, trouvée 530.1580. HPLC (gradient E) *Tr* = 8.56 et 8.81 min (mélange d'isomères 58%-42%).
**C2b**
   Au résidu obtenu lors de la première étape (2,2 mg, 1,5 µmol) ont été ajoutés de l'eau (857 µL), du chlorure de terbium (2,24 mg, 6 µmol) et une solution aqueuse d'hydroxyde de soude 3 M afin d'obtenir une solution pH 6. La solution a été agitée à TA pendant une nuit. L'avancement de la réaction a été suivi par LC-MS (gradient E). Après cette période, la réaction était totale. La solution a été directement purifiée par HPLC préparative (gradient G) et a conduit au complexe **C2b** (231 µg, 10%) identifié comme le composé souhaité. HRMS (ESI+) : calculée pour C₇₂H₈₅N₁₁O₁₇S₂Tb [M+3H]³⁺, *m*/*z* 532.8273, trouvée 532.8268. HPLC (gradient E) *Tr* = 8.71 et 8.94 min (mélange d'isomères 35%-65%).
**C3b :** ce complexe a été préparé selon la même procédure que celle utilisée pour la synthèse de **C1b** et **C2b**.
**Complexes C1c** - **C2c**
   Au composé **26c** (16,3 mg, 11,8 µmol) a été ajouté du TFA (250 µL). La solution a été agitée à TA pendant 2 h. L'avancement de la réaction a été suivi par LC-MS (gradient A). Après cette période, la réaction était totale. Le solvant a été éliminé sous pression réduite. Au résidu ont été ajoutés de l'eau (4 mL) et une solution aqueuse d'hydroxyde de sodium 3 M pour obtenir une solution pH 7. Cette solution a été divisée en deux parties. Une partie a été utilisée pour la préparation du complexe d'europium **C1c** et l'autre partie pour la préparation du complexe de terbium **C2c**.
**C1c**
   A la solution obtenue précédemment (16,3 mg, 11,8 µmol, 3,5 mL), ont été ajoutés du chlorure d'europium (15,1 mg, 41,2 µmol) et une solution aqueuse d'hydroxyde de soude 3 M afin d'obtenir une solution pH 6. La solution a été agitée à TA pendant une nuit. L'avancement de la réaction a été suivi par LC-MS (gradient E). Après cette période, la réaction était totale. La solution a été directement purifiée par HPLC préparative (gradient F) et a conduit au complexe **C1c** (3,25 mg, 18%) identifié comme le composé souhaité. HRMS (ESI+) : calculée pour C₇₀H₇₂N₉O₂,Eu [M+]²⁺, *m*/*z* 763.7027, trouvée 763.7093. HPLC (gradient E) *Tr* = 8.68 min.
**C2c**
   A la solution obtenue précédemment (2,1 mg, 1,5 µmol, 508 µL), ont été ajoutés de l'eau (508 µL), du chlorure de terbium (2,24 mg, 6 µmol) et une solution aqueuse d'hydroxyde de soude 3 M afin d'obtenir une solution pH 6. La solution a été agitée à TA pendant une nuit. L'avancement de la réaction a été suivi par LC-MS (gradient E). Après cette période, la réaction était totale. La solution a été directement purifiée par HPLC préparative (gradient G) et a conduit au complexe **C2c** (184 µg, 8%) identifié comme le composé souhaité. HRMS (ESI+) : calculée pour C₇₀H₇₄N₉O₂₁Tb [M+2H]²⁺, *mlz* 767.7126, trouvée 767.7141. HPLC (gradient E) *Tr =* 8.30 et 8.78 min (mélange d'isomères 25%-75%).
**C3c** : ce complexe a été préparé selon la même procédure que celle utilisée pour la synthèse de **C1c** et **C2c.**
**C1d, C2d, C3d** : ces complexes ont été préparés selon la même procédure que celle utilisée pour la synthèse de **C1c** et **C2c.**
**C1a-maléimide**
   Au complexe de pyridinophane **C1a** (0,56 mg, 400 nmol) ont été ajouté une solution d'hexanoate 6-((béta-maléimidopropionamido) de succinimidyle) (0,23 mg, 600 nmol) dans du DMF anhydre (50 µL) et de la DIPEA (0,2 µL, 155 µg, 1,2 µmol). Le mélange a été agité à TA pendant 2 h. L'avancement de la réaction a été suivi par LC-MS (gradient E) *Tr* = 10.64 min. Après cette période, la réaction était totale. La solution a été directement purifiée par HPLC préparative (gradient H) et a conduit au complexe **C1a-maleimide** (353 µg, 210 nmol, 52%) identifié comme le composé souhaité. LRMS (ESI+) : calculée pour C₇₅H₇₇N₁₁O₂₁S₂Eu [M+H]⁺, *m*/*z* 1684.3933, trouvée 1684.65. HPLC (gradient E) *Tr* = 10.64 min.
**C1a-CAMP**
   Au CAMP-Glu-acide (0,25 mg, 400 nmol) ont été ajoutés du TSTU (0,13 mg, 440 nmol) en solution dans du DMF anhydre (50 µL) et de la DIPEA (0,1 µL, 77,5 µg, 600 nmol). Le mélange a été agité à TA pendant 1 h. Puis à cette solution a été ajouté le complexe **C1a** (0,28 mg, 200 nmol) en solution dans du tampon phosphate 50 mM pH 7 (350 µL). L'avancement de la réaction a été suivi par LC-MS (gradient E) *Tr* = 9,1 min. Après cette période, la réaction était totale. La solution a été directement purifiée par HPLC préparative (gradient H) et a conduit au complexe **C1a-CAMP** (303 µg, 150 nmol, 75%) identifié comme le composé souhaité. LRMS (ESI+) : calculée pour C₈₄H₉₂N₁₆O₂₈PS₂Eu [M+2H]²⁺, *mlz* 1010.2329, trouvée 1010.89. HPLC (gradient E) *Tr* = 9.1 min.
**Composé 27** : disponible commercialement.
**Composé 28** : le composé **28** a été préparé selon la procédure décrite dans EP-A-533131.
**Composé 29**
   A une suspension du composé **28** (2,6 g, 13,4 mmol) dans du chloroforme (265 mL) a été ajouté de la triphénylphosphine (4,2 g, 16,1 mmol). Le mélange a été agité à TA jusqu'à totale dissolution (30 min). A cette solution a été ajouté du tétrachlorure de carbone (37 mL) et le mélange a été agité pendant 20 h à TA. L'avancement de la réaction a été suivi par CCM. Après cette période, la réaction était totale. Le solvant a été éliminé sous pression réduite. Le résidu a été purifié par chromatographie sur colonne de silice en utilisant une éluant cyclohexane - AcOEt 3/7 pour conduire au composé souhaité (0,8 g, 28%) RMN ¹H (CDCl₃, 300 MHz) δ 7.96 (s, 1H), 7.81 (s, 1H), 4.86 (s, 2H), 4.74 (s, 2H), 3.99 (s, 3H), 3.45 (sI 1H).
**Composé 30**
   A une suspension de composé **29** (0,8 g, 3,7 mmol) dans du MeCN anhydre (15 mL) ont été additionnés du carbonate de sodium (1,25 g, 11,8 mmol) et l'amine **18** (250 µL, 1,85 mmol). Le mélange hétérogène a été chauffé à reflux pendant 20 h puis refroidi à TA. A ce mélange ont été additionnés une solution aqueuse saturée de bicarbonate de sodium (20 mL), de l'eau (15 mL) puis du DCM (50 mL). La phase organique a été séparée et la phase aqueuse a été extraite avec du DCM (2 x 50 mL). Les phases organiques ont été réunies, séchées sur sulfate de sodium, filtrées et concentrées sous pression réduite. Le résidu a été purifié par chromatographie sur colonne de silice en utilisant un gradient d'éluant DCM - MeOH 97/3 jusqu'à 90/10 par incrément de 1% pour conduire au composé 30 (0,6 g, 66%). RMN ¹H RMN (CDCl₃, 300 MHz) δ 7,97 (s, 2H), 7,67 (s, 2H), 4.82 (s, 2H), 4.81 (s, 2H), 4.12 (s, 4H), 4.02 (s, 2H), 3.98 (s, 6H), 3.43 (s, 2H), 1.53 (s, 9H).
**Composé 31**
   A une solution de composé diol 30 (50 mg, 102 µmol) dans du DCM (2 mL) ont été additionné de l'iodure de potassium (7 mg, 42,5 µmol) et de l'oxyde d'argent (71 mg, 306 µmol). A ce mélange refroidi à -20°C sous atmosphère inerte a été additionné du TsCI (39 mg, 204 µmol). La solution a été réchauffée à TA puis agitée sous atmosphère inerte pendant 20 h. Après cette période, le mélange réactionnel a été purifié directement par chromatographie sur colonne de silice en utilisant un mélange d'éluant DCM - AcOEt 90/10 pour conduire au dérivé ditosylé **31** sous forme d'une huile incolore (57 mg, 71%). RMN ¹H (CDCl₃) : δ 1,46 (s, 9H) ; 2,42 (s, 6H) ; 3,33 (s, 2H) ; 3,94 (s, 6H) ; 3,96 (s, 4H) ; 5,13 (s, 4H) ; 7,31-7,33 (d, 4H, J = 8,1 Hz); 7,75 (s,2H); 7,80-7,83 (d, 4H, J = 8,4 Hz); 7,96 (s, 2H). RMN ¹³C (CDCl₃): δ = 21,7; 28,2; 52,8; 59,4; 56,1; 71,3; 81,4; 119,3; 122,1; 128,1; 129,9; 132,7; 138,9; 145,2; 154,4; 160,4; 165,3; 170,1. MS (ES+): *m*/*z*= 798,5 [M+H⁺] (100%). IR (cm⁻¹) v 3444, 2958, 1732, 1368, 1177.
**Composé 32**
   Au composé ditosylé **31** (255 mg, 0,319 mmol) en solution dans du MeCN anhydre (160 mL) ont été ajoutés sous argon le composé **23** (204 mg, 0,319 mmol), le carbonate de sodium (338 mg, 3,19 mmol) et l'iodure de sodium (4,8 mg, 0,0319 mmol). La suspension a été agitée à reflux pendant 40 h. Le mélange a été filtré, évaporé sous vide et le résidu a été purifié par chromatographie sur colonne d'alumine (CH₂Cl₂ 100% jusqu'à CH₂Cl₂/MeOH 99/1 par incrément de 0,5%). Les fractions récupérées, correspondant à un mélange de composé sous forme libre et sous forme de complexe de sodium, ont été ensuite resolubilisées dans un minimum de DCM et lavées plusieurs fois à l'eau. Le composé souhaité **32** a été obtenu sous forme d'une poudre jaune (208 mg, 60%). R_{f}= 0,18 (alumine, CH₂Cl₂/MeOH 98/2). RMN ¹H (CDCl₃, 300 MHz) *δ* 7.58 (s, 4H), 7.44 (d, *J* = 8.7 Hz, 2H), 7.16 (s, 2H), 6.86 (d, *J* = 8.7 Hz, 2H), 4.77 (s, 1H), 4.03 (t, *J* = 6 Hz, 2H), 3.95 (s, 4H), 3.92 (s, 4H), 3.84 (s, 4H), 3.79 (s, 6H), 3.43 (s, 2H), 3.39 (s, 4H), 3.35-3.28 (m, 2H), 2.02-1.94 (m, 2H), 1.47 (s, 27H), 1.42 (s, 9H); RMN ¹³C (CDCl₃, 75 MHz) *δ* 170.4, 165.8, 159.6, 159,3, 158.4, 155.9, 137.6, 133.3, 131.8, 122.8, 120.5, 120.3, 114.6, 114.5, 92.8, 86.3, 81.19, 81.15, 79.2, 65.8, 60.0, 59.9, 58.9, 58.7, 52.3, 37.8, 29.5, 28.3, 28.1; HRMS (ESI+): calculée pour C₅₉H₇₈N₇O₁₃ [M+H]⁺, *m*/*z* 1092.5658, trouvée 1092.5636; IR (cm⁻¹): *v* 3429, 2977, 2932, 2210, 1729, 1596, 1511, 1367, 1225, 1159.
**Composé 34**
   Dans un ballon de 25 mL le composé **32** (50,0 mg, 46 µmol) a été solubilisé dans MeCN (1 mL) pour donner une solution incolore. Au mélange réactionnel a été ajouté l'hydroxyde de lithium (11,19 mg, 458 µmol) en solution dans de l'eau (458 µL) en une seule fois. La solution a été agitée à TA pendant 30 min. L'avancement de la réaction a été suivi par UPLC-MS (gradient I). Après cette période, la réaction était totale. La solution a été directement purifiée par HPLC préparative (gradient J) et a conduit au composé **34** (35 mg, 71%) identifié comme le composé souhaité. HRMS (ESI+) : calculée pour C₅₇H₇₄N₇O₁₃ [M+H]⁺, *m*/*z* 1064.5339, trouvée 1064.5341. UPLC-MS (gradient I) *Tr* = 3.82 min.
**Composé 35a**
   Dans un ballon de 50 mL le composé **34** (35,0 mg, 33 µmol) a été solubilisé dans un mélange DMSO (1 mL) et MeCN (4 mL) pour donner une solution incolore. Au mélange réactionnel a été ajouté la DIPEA (34 µl, 197 µmol), la taurine (12,47 mg, 99 µmol) et le HATU (25,8 mg, 65,8 µmol) en une seule fois. La solution a été agitée à TA pendant 2 h. L'avancement de la réaction a été suivi par UPLC-MS (gradient I). Après cette période, la réaction était totale. La solution a été directement purifiée par HPLC préparative (gradient K) et a conduit au composé **35a** (31,7 mg, 75 %) identifié comme le composé souhaité. HRMS (ESI+) : calculée pour C₆₁H₈₅N₉O₁₇S₂ [M+2H]²⁺, *m*/*z* 639.7747, trouvée 639.7747. UPLC-MS (gradient I) *Tr* = 3.36 min.
**Composé 35b**
   Le 3-((2-aminoéthyl)diméthylammonio)propane-1-sulfonate a été préparé selon la procédure décrite dans WO 2011/146595 et dans Organic & Biomolecular Chemistry 2012, 10, 1883. Dans un ballon de 5 mL le 3-((2-aminoéthyl)diméthylammonio)propane-1-sulfonate (10,56 mg, 50,0 µmol) et le composé **34** (10,64 mg, 10 µmol) ont été solubilisés dans du DMSO (600 µL) pour donner une solution jaune. Au mélange réactionnel a été ajouté la DIPEA (12,06 µL, 70,0 µmol) et le HATU (15,21 mg, 40,0 µmol) en une seule fois. La solution a été agitée à TA pendant 15 min. L'avancement de la réaction a été suivi par UPLC-MS (gradient I). Après cette période, la réaction était totale. La solution a été directement purifiée par HPLC préparative (gradient L) et a conduit au composé **35b** (8,6 mg, 59%) identifié comme le composé souhaité. HRMS (ESI+) : calculée pour C₇₁H₁₀₇N₁₁O₁₇S₂ [M+2H]²⁺, *m*/*z* 724.8638, trouvée 724.8644. UPLC-MS (gradient I) *Tr* = 3.24 min.
**Composé 35c** : ce composé a été préparé selon la même procédure que celle utilisée pour la synthèse de **35a** et **35b.**
**Composé 35d**
   L'hydrochlorure de 2-triméthylammonium-éthylamine a été préparé selon la procédure décrite dans Analytical Chemistry 2014, 86, 10006. Dans un ballon de 5 mL l'hydrochlorure de 2-triméthylammonium-éthylamine (6,98 mg, 50,0 µmol) et le composé **34** (10,64 mg, 10 µmol) ont été solubilisés dans le DMSO (600 µL) pour donner une solution jaune. Au mélange réactionnel a été ajouté la DIPEA (16,68 µL, 100 µmol) et le HATU (15,21 mg, 40,0 µmol) en une seule fois. La solution a été agitée à TA pendant 15 min. L'avancement de la réaction a été suivi par UPLC-MS (gradient I). Après cette période, la réaction était totale. La solution a été directement purifiée par HPLC préparative (gradient L) et a conduit au composé 35d (6,8 mg, 55%) identifié comme le composé souhaité. HRMS (ESI+) : calculée pour C₆₇H₉₉N₁₁O₁₁ [M+2H]²⁺, *m*/*z* 616.8757, trouvée 616.8759. UPLC-MS (gradient I) *Tr* = 2.97 min.
**Complexe C4a**
   Dans un ballon de 50 mL le composé **35a** (31,7 mg, 25 µmol) a été solubilisé dans du TFA (200 µL) pour donner une solution jaune. La solution a été agitée à TA pendant 30 min. L'avancement de la déprotection a été suivi par UPLC-MS (gradient I). Après cette période, la déprotection était totale. Le TFA a été évaporé sous pression réduite. Le résidu a été solubilisé dans de l'eau (6 mL) et MeCN (1 mL) en ajustant le pH à 7 avec une solution de soude 3 M. Au mélange réactionnel sous agitation a été ajouté le chlorure d'europium(III) hexahydrate (36,3 mg, 99 µmol) en une seule fois. Le pH a été ajusté à 7 par ajout de soude 3 M. La solution a été agitée à TA pendant 5 j. L'avancement de la réaction a été suivi par UPLC-MS (gradient I). Après cette période, la réaction était totale. La solution a été directement purifiée par HPLC préparative (gradient M) et a conduit au complexe C4a (15,6mg, 55%) identifié comme le composé souhaité. HRMS (ESI+) : calculée pour C₄₄H₅₀N₉O₁₅S₂Eu [M+2H]²⁺, *m*/*z* 579.6028, trouvée 579.6026. UPLC-MS (gradient N), isomère 1 *Tr* = 1.32 min et isomère 2 *Tr* = 1.64 min.
**Complexe C5a :** ce complexe a été préparé selon la même procédure que celle utilisée pour la synthèse de **C4a.**
**Complexe C4b**
   Dans un ballon de 25 mL le composé **35b** (8,6 mg, 5,93 µmol) a été solubilisé dans du TFA (130 µL) pour donner une solution jaune. La solution a été agitée à TA pendant 30 min. L'avancement de la déprotection a été suivi par UPLC-MS (gradient I). Après cette période, la déprotection était totale. Le TFA a été évaporé sous pression réduite. Le résidu a été solubilisé dans du tampon HEPES 50 mM pH 7,4 (2 mL). Au mélange réactionnel sous agitation a été ajouté le chlorure d'europium(III) hexahydrate (13,03 mg, 36 µmol) en une seule fois. La solution a été agitée à TA pendant 40 h. L'avancement de la réaction a été suivi par UPLC-MS (gradient I). Après cette période, la réaction était totale. La solution a été directement purifiée par HPLC préparative (gradient M) et a conduit au complexe **C4b** (6,5 mg, 83%) identifié comme le composé souhaité. HRMS (ESI+) : calculée pour C₅₄H₇₃N₁₁O₁₅S₂Eu [M+3H]³⁺, *m*/*z* 444.1310, trouvée 444.1309. UPLC-MS (gradient I) *Tr* = 0.34 min.
**Complexe C5b** : ce complexe a été préparé selon la même procédure que celle utilisée pour la synthèse de **C4b.**
**Complexe C4c :** ce complexe a été préparé selon la même procédure que celle utilisée pour la synthèse de **C4b.**
**Complexe C5c** : ce complexe a été préparé selon la même procédure que celle utilisée pour la synthèse de **C4b.**
**Complexe C4d**
   Dans un ballon de 25 mL le composé **35d** (6,8 mg, 5,51 µmol) a été solubilisé dans du TFA (200 µL) pour donner une solution jaune. La solution a été agitée à TA pendant 5 h. L'avancement de la déprotection a été suivi par UPLC-MS (gradient I). Après cette période, la déprotection était totale. Le TFA a été évaporé sous pression réduite. Le résidu a été solubilisé dans du tampon HEPES 50 mM pH 7,4 (2 mL). Au mélange réactionnel sous agitation a été ajouté le chlorure d'europium(III) hexahydrate (12,09 mg, 33 µmol) en une seule fois. La solution a été agitée à TA pendant 18 h. L'avancement de la réaction a été suivi par UPLC-MS (gradient I). Après cette période, la réaction était totale. La solution a été directement purifiée par HPLC préparative (gradient M) et a conduit au complexe **C4d** (3 mg, 19%) identifié comme le composé souhaité. HRMS (ESI+) : calculée pour C₅₀H₆₄N₁₁O₉Eu [M+2H]²⁺, *m*/*z* 557.7048, trouvée 557.7050. UPLC-MS (gradient I)*Tr* = 0.32 min. **Complexe C5d** : ce complexe a été préparé selon la même procédure que celle utilisée pour la synthèse de **C4b.**
**Complexe C4e :** ce complexe a été préparé selon la même procédure que celle utilisée pour la synthèse de **C4b.**
**Complexe C5e :** ce complexe a été préparé selon la même procédure que celle utilisée pour la synthèse de **C4b.**
**Composé 37**
   Le composé **37** a été préparé selon la procédure décrite dans l'article Chemistry - A European Journal 2008, 14, 1726. L'acide chélidamique monohydraté **5** (3 g, 15 mmol) a été chauffé à reflux dans EtOH (60 mL) en présence d'acide sulfurique (0,6 mL) à 97% pendant 16 h. Le solvant a été évaporé sous pression réduite, le résidu blanc restant a été neutralisé avec une solution aqueuse saturée de bicarbonate de sodium et extrait avec du DCM. La phase organique a été séchée avec du sulfate de magnésium, évaporée à sec et le diester **37** a été obtenu sous forme d'un solide blanc (2,27 g, 63%). R_{f} = 0.10 (silice, CH₂Cl₂/MeOH 99/1) ; RMN ¹H (CDCl₃, 300 MHz) *δ* 7.31 (s, 2H), 4.46 (q, *J* = 7.1 Hz, 4H), 1.42 (t, *J* = 7.1 Hz, 6H); RMN ¹³C (DMSO-d₆, 62.5 MHz) *δ* 166.0, 164.3, 149.9, 115.2, 61.4, 14.1.
**Composé 38**
   Le composé **38** a été préparé selon la procédure décrite dans l'article Organic & Biomolecular Chemistry 2012, 10, 9183. A une solution de diéthyl chélidamate **37** (0,5 g, 2,09 mmol) dans du THF anhydre (50 mL) ont été ajoutés sous argon de la triphénylphosphine (1,13 g, 4,31 mmol) et du *tert*-butyl-(3-hydroxypropyl) carbamate (0,77g, 4,41mmol) dilué dans du THF anhydre (10 mL). Du DIAD (0,83 mL, 4,19 mmol) a été ensuite ajouté goutte à goutte pendant 10 min et le milieu réactionnel a été agité à 70°C pendant 16 h. Le milieu a été évaporé sous pression réduite et le résidu visqueux jaune obtenu a été purifié par chromatographie flash sur colonne de silice (AcOEt/EP 50/50). Le composé **38** a été obtenu sous forme d'une poudre blanche (0,82 g, 99%). R_{f} = 0,23 (silice, AcOEt/EP 50/50); RMN ¹H (CDCl₃, 300 MHz) *δ* 7.77 (s, 2H), 4.68 (s, 1H), 4.47 (q, *J* = 7.1 Hz, 4H), 4.19 (t, *J* = 5.9 Hz, 2H), 3.39-3.28 (m, 2H), 2.10-1.99 (m, 2H), 1.45 (t, *J* = 7.1 Hz, 6H), 1.43 (s, 9H); RMN ¹³C (CDCl₃, 75 MHz) *δ* 166.8, 164.7, 156.0, 150.2, 114.3, 79.4, 66.6, 62.4, 37.5, 29.4, 28.4, 14.2.
**Composé 39**
   Le composé **39** a été préparé selon la procédure décrite dans l'article Organic & Biomolecular Chemistry 2012, 10, 9183. A une solution de composé **38** (1,70 g, 4,30 mmol) dans EtOH (85 mL) a été ajouté du borohydure de sodium (817 mg, 21,595 mmol) en petites portions. Le milieu réactionnel a été chauffé à reflux pendant 2 h. Le mélange a été ensuite refroidi à TA et 70 mL d'eau ont été ajoutés et l'éthanol a été éliminé par sous pression réduite. La phase aqueuse a été extraite avec du DCM (4 x 20 mL). La phase organique a été lavée avec une solution saturée de chlorure d'ammonium (20 mL) puis avec de l'eau (20 mL), séchée avec du sulfate de magnésium, et évaporée sous pression réduite. Le composé 39 est alors obtenu sous forme d'un solide blanc (1,02 g, 76%). R_{f} = 0.30 (silice, CH₂Cl₂/MeOH 90/10); RMN ¹H (CDCl₃, 300 MHz) *δ* 6.72 (s, 2H), 4.71 (s, 4H), 4.68 (s, 1H), 4.09 (t, J=6.1 Hz, 2H), 3.37-3.23 (m, 2H), 2.77 (s, 2H), 2.07-1.93 (m, 2H), 1.43 (s, 9H); RMN ¹³C (CDCl₃, 75 MHz) *δ* 166.5, 161.2, 156.3, 105.6, 79.4, 65.7, 64.4, 37.6, 29.4, 28.5.
**Composé 40**
   Le composé **40** a été préparé selon la procédure décrite dans l'article Organic & Biomolecular Chemistry 2012, 10, 9183. A une solution du composé **39** (440 mg, 1,41 mmol) dans un mélange THF/eau (1:1,17 mL) à 0°C sous agitation ont été ajouté de la soude (338 mg, 8,45 mmol) et une solution de TsCI (1,07 g, 5,64 mmol) dans du THF (23 mL). Le milieu réactionnel a été agité à TA pendant 16 h, puis décanté, et la phase aqueuse a été lavée avec du DCM (10 mL). Les phases organiques rassemblées ont été lavées avec une solution aqueuse à 5% de bicarbonate de sodium (10 mL), séchées avec sulfate de magnésium, filtrées et évaporées à sec. Le composé **40** a été obtenu sous forme d'une poudre blanche (841 mg, 96%). R_{f} = 0,40 (silice, CH₂Cl₂/MeOH 98/2); RMN ¹H (CDCl₃, 300 MHz) *δ* 7.80 (d, *J* = 8.0 Hz, 4H), 7.33 (d, *J* = 8.0Hz, 4H), 6.81 (s, 2H), 4.98 (s, 4H), 4.68 (s, 1H), 4.03 (t, *J* = 6.0 Hz, 2H), 3.36-3.23 (m, 2H), 2.44 (s, 6H), 2.06-1.91 (m, 2H), 1.45 (s, 9H); RMN ¹³C (CDCl₃, 75 MHz) *δ* 166.7, 156.1, 153.3, 145.3, 132.8, 130.1, 128.2, 107.7, 79.6, 71.3, 66.1, 37.6, 29.4, 28.5, 21.8.
**Composé 41**
   A une solution du composé **19** (943 mg, 2,98 mmol) dans du DMF anhydre (26 mL) sous argon ont été ajouté le carbonate de césium (1,01 g, 3,12 mmol) et le composé **40** (844 mg, 1,36 mmol). Le milieu réactionnel a été agité à TA pendant 40 h, filtré, évaporé à sec et le produit brut obtenu a été purifié par chromatographie sur colonne de silice (CH₂Cl₂ 100% puis CH₂Cl₂/AcOEt 90/10) pour conduire au composé **41** sous forme d'une poudre blanche (980 mg, 79%). R_{f} = 0,58 (silice, CH₂Cl₂/AcOEt 90/10); RMN ¹H (CDCl₃, 300 MHz) *δ* 8.11-8.04 (m, 2H), 7.72-7.60 (m, 6H), 6.76 (s, 2H), 4.70 (s, 1H), 4.60 (s, 4H), 4.12 (s, 4H), 4.02-3.92 (m, 2H), 3.35-3.21 (m, 2H), 2.03-1.87 (m, 2H), 1.45 (s, 9H), 1.35 (s, 18H); RMN ¹³C (CDCl₃, 75 MHz) *δ* 167.4, 166.6, 157.2, 155.9, 147.9, 133.58, 133.55, 131.8, 130.9, 124.1, 107.6, 82.3, 79.4, 65.7, 53.2, 49.1, 37.5, 29.2, 28.4, 27.8; LRMS (ESI+) : calculée pour C₃₉H₅₃N₆O₁₅S₂ [M+H]⁺, *m*/*z* 909.30, trouvée 909.30.
**Composé 42**
   A une solution de composé di-nosylé **41** (1,12 g, 1,23 mmol) dans MeCN (54 mL) ont été ajoutés sous agitation du 2-mercaptoéthanol (949 µL, 13,574 mmol) et du 1.8-diazabicyclo [5-4-0] unde-7-ène (828 µL, 5,55 mmol). Le mélange a été agité pendant 30 min à TA. Le solvant a été éliminé sous pression réduite puis du DCM (20 mL) a été ajouté et le mélange a été lavé avec de l'eau (2 x 10 mL), séché avec du sulfate de magnésium, filtré et évaporé sous pression réduite. Le produit brut obtenu a été purifié par chromatographie sur colonne de silice (AcOEt/MeOH 95/5 puis 90/10) pour conduire au composé **42** sous forme d'un solide jaune (523 mg, 79%). R_{f} = 0,24 (silice, AcOEt/MeOH 90/10); RMN ¹H (CDCl₃, 300 MHz) *δ* 6.73 (s, 2H), 4.79-4-75 (m, 1H), 4.04 (t, J = 6 Hz, 2H), 3.84 (s, 4H), 3.73 (s, 2H), 3.34 (s, 4H), 3.31-3.25 (m, 2H), 2.00-1.92 (m, 2H), 1.44 (s, 18H), 1.42 (s, 9H); RMN ¹³C (CDCl₃, 75 MHz) *δ* 171.4, 166.1, 160.5, 156.0, 106.7, 81.2, 79.3, 65.5, 54.5, 51.2, 37.6, 29.3, 28.4, 28.1; MS (ESI+) calculée pour C₂₇H₄₇N₄O₇ [M+H]⁺, *m*/*z* 539.3445, trouvée 539.3445.
**Composé 43**
   A une solution de composé **12** (945 mg, 0,893 mmol) dans MeCN (447 mL) ont été ajoutés sous argon le composé **42** (480 mg, 0,893 mmol), du carbonate de sodium (947 mg, 8,93 mmol) et de l'iodure de sodium (13 mg, 0,0893 mmol). La suspension a été agitée à reflux pendant 65 h. Le résidu a été filtré, évaporé sous pression réduite et purifié par chromatographie sur colonne d'alumine (DCM 100% jusqu'à DCM/MeOH 95/5 par incrément de 1%). Les fractions récupérées, correspondant à un mélange de composé sous forme libre et sous forme de complexe de sodium, ont été ensuite resolubilisées dans un minimum de DCM et lavées plusieurs fois à l'eau. Le composé souhaité **43** a été obtenu sous forme d'une poudre jaune (690 mg, 62%). R_{f} = 0,33 (alumine, DCM/MeOH 97/3); RMN ¹H (CDCl₃, 300 MHz) δ 7.40 (d, *J* = 8.8 Hz, 4H), 7.24 (d, *J* = 1.5 Hz, 2H), 7.20 (d, *J* = 1.5 Hz, 2H), 6.79 (d, *J* = 8.8 Hz, 4H), 6.64 (s, 2H), 4.71 (s, 1H), 4.64 (s, 4H), 4.01-3.96 (m, 2H), 3.86-3.83 (m, 12H), 3.81 (s, 6H), 3.39 (s, 6H), 3.17-3.08 (m, 2H), 1.82-1.70 (m, 2H), 1.48 (s, 9H), 1.46 (s, 18H), 1.41 (s, 9H); RMN ¹³C (CDCl₃, 75 MHz) *δ* 170.5, 168.9, 166.2, 158.5, 158.1, 155.9, 133.4, 131.7, 123.2, 122.9, 115.6, 114.7, 107.9, 92.6, 86.6, 81.2, 81.1, 79.0, 65.8, 65.1, 60.1, 59.8, 59.6, 58.31, 58.28, 52.3, 37.5, 29.6, 28.4, 28.2. MS (ESI+): *m*/*z* 626.8 [M+2H]²⁺, 42%, 645.8 [M+K+H]²⁺, 100%), 1252.6 ([M+H]⁺, 15%); HRMS (ESI+) calculée pour C₆₉H₈₆N₇O₁₅ [M+H]⁺, *m*/*z* 1252.6182, trouvée 1252.6194; IR (cm⁻¹): v 3425, 2976, 2931, 2210, 1758, 1733, 1596, 1510, 1367, 1213, 1158.
**Composé 44**
   Dans un ballon de 5 mL le composé **43** (27,0 mg, 22 µmol) a été solubilisé dans MeCN (1 mL) pour donner une solution incolore. Au mélange réactionnel a été ajouté de l'hydroxyde de lithium (2,66 mg, 109 µmol) en solution dans de l'eau (500 µL) en une seule fois. La solution a été agitée à TA pendant 30 min. L'avancement de la réaction a été suivi par UPLC-MS (gradient I). Après cette période, la réaction était totale. La solution a été directement purifiée par HPLC préparative (gradient O) et a conduit au composé **44** (22,9 mg, 86%) identifié comme le composé souhaité. HRMS (ESI+) : calculée pour C₆₇H₈₂N₇O₁₅ [M+H]⁺, *m*/*z* 1224.5863, trouvée 1224.5867. UPLC-MS (gradient I) *Tr* = 3.87 min.
**Composé 45a**
   Dans un ballon de 25 mL le composé **44** (22,90 mg, 19 µmol) a été solubilisé dans du DMSO (1 mL) pour donner une solution incolore. Au mélange réactionnel ont été ajouté la taurine (11,82 mg, 94 µmol), la DIPEA (22 µl, 131 µmol) puis le HATU (29,3 mg, 74,8 µmol) en une seule fois. La solution a été agitée à TA pendant 2 h. L'avancement de la réaction a été suivi par UPLC-MS (gradient I). Après cette période, la réaction était totale. La solution a été directement purifiée par HPLC préparative (gradient K) et a conduit au composé **26a** (27 mg, 70%) identifié comme le composé souhaité. HRMS (ESI+) : calculée pour C₇₁H₉₃N₉O₁₉S₂ [M+2H]²⁺, *m*/*z* 719.8009, trouvée 719.8011. UPLC-MS (gradient I) *Tr* = 3.22 min.
**Composé 45b** : ce composé a été préparé selon la même procédure que celle utilisée pour la synthèse de **45a** en sélectionnant l'amine correspondante.
**Composé 45c** : ce composé a été préparé selon la même procédure que celle utilisée pour la synthèse de **45a** en sélectionnant l'amine correspondante.
**Composé 45c :** ce composé a été préparé selon la même procédure que celle utilisée pour la synthèse de **45a** en sélectionnant l'amine correspondante.
**Composé 45d** : ce composé a été préparé selon la même procédure que celle utilisée pour la synthèse de 45a en sélectionnant l'amine correspondante.
**Complexe C6a** :
   Dans un ballon de 10 mL le composé **45a** (18,9 mg, 13 µmol) a été solubilisé dans du TFA (200 µL) pour donner une solution jaune. La solution a été agitée à TA pendant 30 min. L'avancement de la déprotection a été suivi par UPLC-MS (gradient I). Après cette période, la déprotection était totale. Le TFA a été éliminé sous pression réduite. Le résidu a été solubilisé dans du tampon HEPES 50 mM pH 7,4 (6,2 mL). Au mélange réactionnel sous agitation a été ajouté le chlorure d'europium(III) hexahydrate (27,5 mg, 75 µmol) en une seule fois. La solution a été agitée à TA pendant 4 j. L'avancement de la réaction a été suivi par UPLC-MS (gradient I). Après cette période, la réaction était totale. La solution a été directement purifiée par HPLC préparative (gradient M) et a conduit au complexe **C6a** (0,89 mg, 5%) identifié comme le composé souhaité. HRMS (ESI+) : calculée pour C₅₄H₅₈N₉O₁₇S₂Eu [M+2H]²⁺, *m*/*z* 660.6299, trouvée 660.6299. UPLC-MS (gradient I), isomère 1 *Tr* = 1,5 min et isomère 2 *Tr* = 1,55 min.
**Complexe C7a :** ce complexe a été préparé selon la même procédure que celle utilisée pour la synthèse de **C6a.**
**Complexe C7a :** ce complexe a été préparé selon la même procédure que celle utilisée pour la synthèse de **C6a.**
**Complexe C6b :** ce complexe a été préparé selon la même procédure que celle utilisée pour la synthèse de **C6a.**
**Complexe C7b :** ce complexe a été préparé selon la même procédure que celle utilisée pour la synthèse de **C6a.**
**Complexe C6c :** ce complexe a été préparé selon la même procédure que celle utilisée pour la synthèse de **C6a.**
**Complexe C6d** : ce complexe a été préparé selon la même procédure que celle utilisée pour la synthèse de **C6a.**
**Complexe C7d :** ce complexe a été préparé selon la même procédure que celle utilisée pour la synthèse de **C6a.**
**Composé 46** : disponible commercialement.
**Composé 47** : le composé **47** a été préparé selon la procédure décrite dans Journal Organic Chemistry 1987, 52, 2029.
**Composé 48 :** le composé **48** a été préparé selon la procédure décrite dans EP2216330.
**Composé 49** : le composé **49** a été préparé selon la procédure décrite dans EP2216330 pour un composé analogue.
**Composé 50** : le composé **50** a été préparé selon la procédure décrite dans EP2216330 pour un composé analogue.
**Composé 51:** ce composé a été préparé selon la même procédure que celle utilisée pour la synthèse de **15.**
**Composé 52 :** le composé **52** a été préparé selon la procédure décrite dans Organic Letters 2014, 16, 1290.
**Composé 53:** ce composé a été préparé selon la même procédure que celle utilisée pour la synthèse de **20.**
**Composé 54:** ce composé a été préparé selon la même procédure que celle utilisée pour la synthèse de **21.**
**Composé 55:** ce composé a été préparé selon la même procédure que celle utilisée pour la synthèse de **23.**
**Composé 56:** ce composé a été préparé selon la même procédure que celle utilisée pour la synthèse de **9.**
**Composé 57:** ce composé a été préparé selon la même procédure que celle utilisée pour la synthèse de **10.**
**Composé 58:** ce composé a été préparé selon la même procédure que celle utilisée pour la synthèse de **12.**
**Composé 59:** ce composé a été préparé selon la même procédure que celle utilisée pour la synthèse de **24.**
**Composé 60:** ce composé a été préparé selon la même procédure que celle utilisée pour la synthèse de **25.**
**Composé 61a-d:** ces composés ont été préparés selon la même procédure que celle utilisée pour la synthèse de **26a-d.**
**Complexes C8a-d:** ces complexes ont été préparés selon la même procédure que celle utilisée pour la synthèse de **C1a-d**.
**Complexes C9a-d:** ces complexes ont été préparés selon la même procédure que celle utilisée pour la synthèse de **C2a-d.**
**Complexes C10a-d:** ces complexes ont été préparés selon la même procédure que celle utilisée pour la synthèse de **C3a-d.**

### Mesures photo-physiques

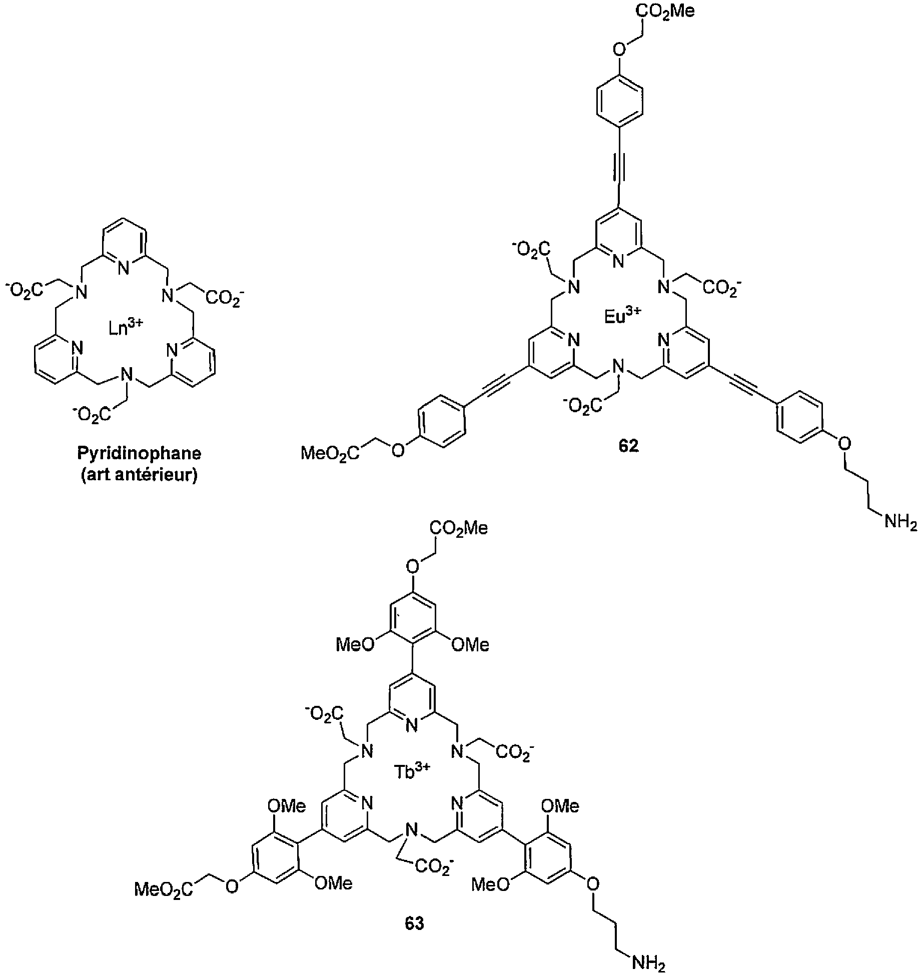

Les propriétés photophysiques de complexes représentatifs de l'invention ont été reportées dans le tableau ci-dessous.

**Tableau 1 : Propriétés photo-physiques des complexes**

| **Complexe** | ***Lanthanide* (Ln)** | ***Nbre Chrom°*** | ***Groupe soluble*** | ***Absorption** λ*max (nm) | ***Brillance** ε*x Φ (H₂O) | ***Durée de vie*** *τ*_{H₂O} (ms) | ***Solubilité dans H₂O*** |
|---|---|---|---|---|---|---|---|
| **Pyridinophane** | Eu | 0 | - | 267 | 564 | 0.78 | oui |
| **62** | Eu | 3 | - | 320 | - | - | non |
| **63** | Tb | 3 | - | 328 | - | - | non |
| **C1a** | Eu | 3 | Sulfo | 320 | 5100 | 0.74 | oui |
| **C1b** | Eu | 3 | Sulfobétaïne | 320 | 6000 | 0.68 | oui |
| **C1c** | Eu | 3 | Sucre | 318 | 6000 | 0.70 | oui |
| **C4a** | Eu | 1 | Sulfo | 320 | 1400 | 0.69 | oui |
| **C4b** | Eu | 1 | Sulfobétaïne | 321 | 1400 | 0.73 | oui |
| **C4d** | Eu | 1 | Ammonium | 320 | 1400 | 0.76 | oui |

| | | | | | | | |
|---|---|---|---|---|---|---|---|
| a : Chrom = chromophore | | | | | | | |

Le pyridinophane (art antérieur) de base est soluble dans l'eau (milieux biologiques et tampons) mais le chromophore (pyridine) n'est pas adapté à une excitation entre 310-350 nm (excitation par lampe flash ou laser à azote) puisque l'absorption maximale de ce complexe est de 267 nm. L'introduction des chromophores sur ces systèmes pyridinophanes sans groupement solubilisant (composé **62, 63**) rendent ces complexes compatibles avec les sources d'excitation laser ou flash (absorption **62** et **63** autour de 320-330 nm) mais ces complexes sont insolubles dans l'eau (milieux biologiques et tampons). Cette absence de solubilité interdit toute utilisation de **62** et **63** dans un immunoessai de type HTRF. En revanche dès que des groupements solubilisants sont introduits sur **62** et **63** correspondants aux complexes de l'invention (exemples **C1a-c** ou **C4a-b, C4d)** alors ces composés deviennent solubles dans les milieux biologiques et tampon et les complexes peuvent être utilisés dans un immunoessai de type HTRF, en microscopie ou autres applications des sciences de la vie.

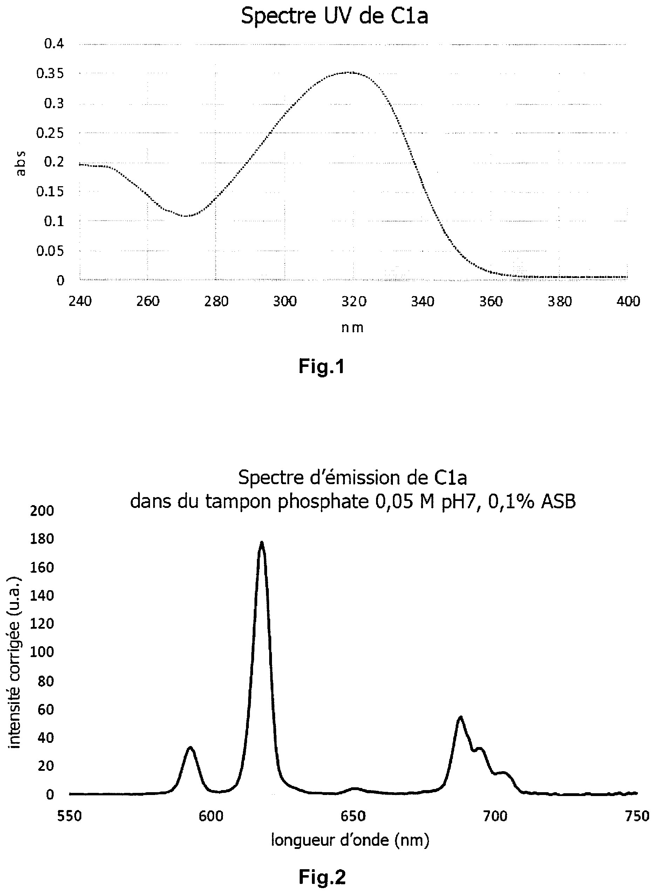

Les figures 1 et 2 montrent les spectres d'absorption et d'émission du complexe C1a. Le spectre d'absorption présente un maximum à 320 nm. Ainsi cette série de complexe est parfaitement compatible avec une excitation laser et lampe flash. Le spectre d'émission présente un maximum à 620 nm ce qui permet d'avoir un recouvrement optimal avec un accepteur compatible.

## Revendications

1. Agent complexant de formule (I) 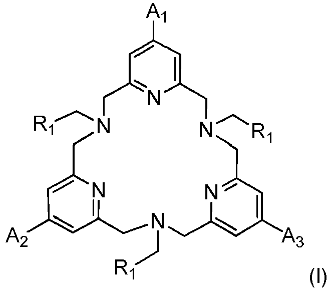 dans laquelle :
- les groupes R₁ sont identiques et sont choisis parmi : -CO₂H, -PO(OH)R, R étant choisi parmi les groupes: phényle, phényle substitué par un groupe -SO₃H, de préférence en position ortho ou para, benzyle, méthyle, éthyle, propyle, n-butyle, *sec-*butyle, isobutyle, *tert*-butyle;
- les groupes A₁, A₂ sont identiques ou différents et sont choisis parmi : un groupe de formule -L₁-E ; un groupe de formule (II) ou (II') ;
- le groupe A₃ est choisi parmi : un groupe de formule -O-L₃-G, un groupe de formule (II), (II'), (III) ou (III') ; 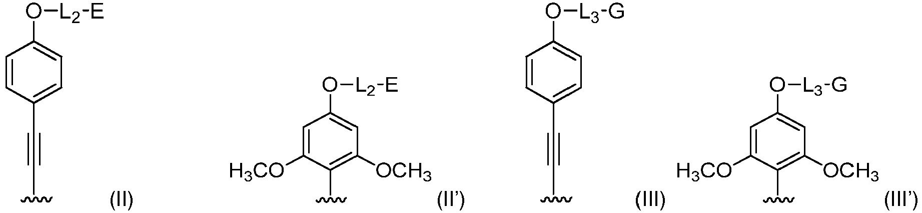 L₁, L₂ et L₃ sont identiques ou différents et représentent un groupe de liaison divalent choisi parmi :
- un groupe alkylène linéaire ou ramifié en C₁-C₂₀, contenant éventuellement une ou plusieurs doubles ou triples liaisons;
- un groupe cycloalkylène en C₅-C₈ ou un groupe arylène en C₆-C₁₄,
lesdits groupes alkylène, cycloalkylène ou arylène contenant éventuellement un ou plusieurs hétéroatomes, tels que l'oxygène, l'azote, le soufre, le phosphore ou un ou plusieurs groupe(s) carbamoyle ou carboxamido, et lesdits groupes alkylène, cycloalkylène ou arylène étant éventuellement substitués par des groupes alkyle en C₁-C₈, aryle en C₆-C₁₄, sulfonate ou oxo ;
- un groupe choisi parmi les groupes divalents de formules suivantes : 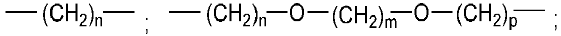 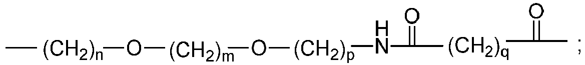 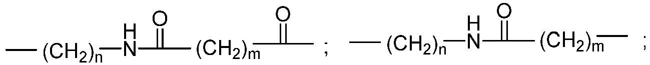 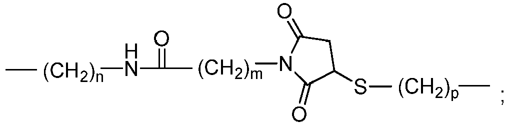 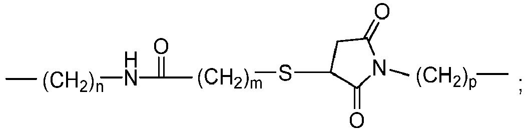 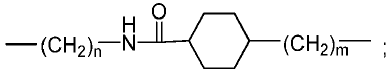 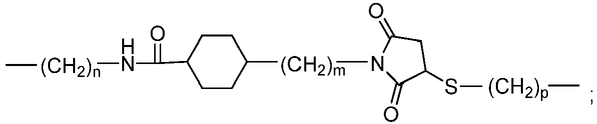 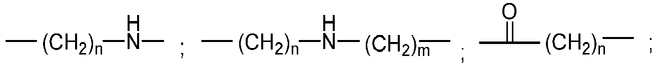 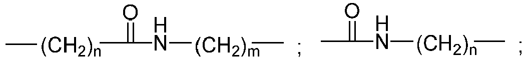 dans lesquelles n, m, p, q sont des nombres entiers de 1 à 16, de préférence de 1 à 5 ;
- E est un groupe qui rend l'agent complexant hydrosoluble, choisi parmi : -SO₃H, -PO(OH)₂, -CO₂H, -N⁺Alk₁Alk₂Alk₃, un résidu glucose sous forme cyclique ou linéaire, ou bien un groupe de formule -(CHOH)ₖ-CH₂OH, k étant un nombre entier allant de 3 à 12; une sulfobétaïne ; un groupe PEG de formule -CH₂-(CH₂OCH₂)_{y}-CH₂OCH₃, y étant un nombre entier allant de 1 à 5 ;
- G est un groupe réactif choisi parmi : un acrylamide, une amine activée, un ester activé, un aldéhyde, un halogénure d'alkyle, un anhydride, une aniline, un azide, une aziridine, un acide carboxylique, un diazoalcane, un haloacétamide, une halotriazine, la dichlorotriazine, une hydrazine, un imido ester, un isocyanate, un isothiocyanate, un maléimide, un halogénure de sulfonyle, un thiol, une cétone, une amine, un halogénure d'acide, un ester de succinimidyle, un ester d'hydroxysuccinimidyle, un ester d'hydroxysulfosuccinimidyle, un azidonitrophényle, un azidophényle, un glyoxal, une triazine, un groupe acétylénique, et les groupes de formule : 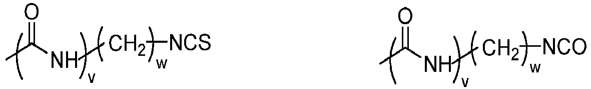 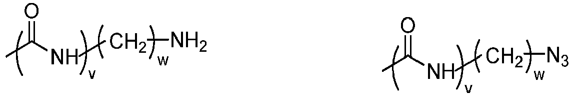 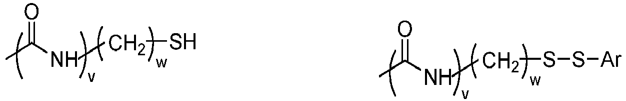 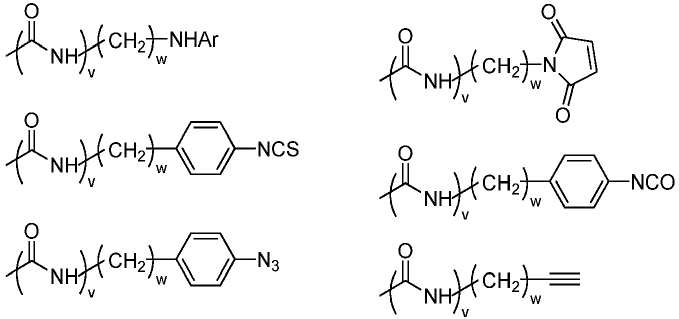 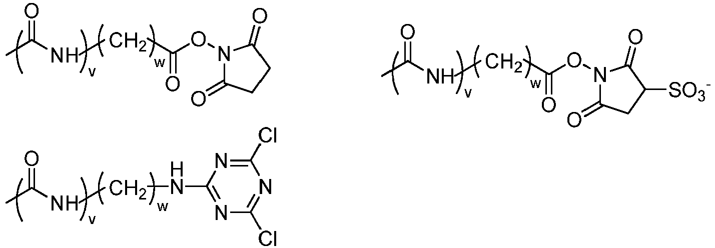 dans lesquels w varie de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène ;
- Alk₁, Alk₂, Alk₃, qui peuvent être identiques ou différents, représentent un (C₁-C₆)alkyle.

2. Agent complexant selon la revendication 1, **caractérisé en ce que** A₁ et A₂ sont identiques et sont un groupe de formule (II) et A₃ est un groupe de formule (III).

3. Agent complexant selon la revendication 1, **caractérisé en ce que** A₁ est un groupe de formule (II), A₂ est un groupe -L₁-E et A₃ est un groupe de formule (III).

4. Agent complexant selon la revendication 1, **caractérisé en ce que** A₁ et A₂ sont identiques et sont des groupes de formule (II), et A₃ est un groupe de formule -O-L₃-G.

5. Agent complexant selon la revendication 1, **caractérisé en ce que** A₁ et A₂ sont identiques et sont des groupes de formule -L₁-E et A₃ est un groupe de formule (III).

6. Agent complexant selon la revendication 1, **caractérisé en ce que** A₁ et A₂ sont identiques et sont des groupes de formule (II') et A₃ est un groupe de formule (III').

7. Agent complexant selon la revendication 1, **caractérisé en ce que** A₁ est un groupe de formule (II'), A₂ est un groupe -L-E et A₃ est un groupe de formule (III').

8. Agent complexant selon la revendication 1, **caractérisé en ce que** A₁ et A₂ sont identiques et sont des groupes de formule (II'), et A₃ est un groupe de formule -O-L₃-G.

9. Agent complexant selon la revendication 1, **caractérisé en ce que** A₁ et A₂ sont identiques et sont des groupes de formule -L₁-E et A₃ est un groupe de formule (III').

10. Agent complexant selon l'une quelconque des revendications précédentes, **caractérisé en ce que** le groupe -L₃-G, lorsqu'il est présent, est constitué d'un groupement réactif G choisi parmi : un acide carboxylique, une amine, de préférence une amine aliphatique, un ester de succinimidyle, un haloacétamide, une hydrazine, un isothiocyanate, un groupe maléimide, et d'un bras d'espacement L₃ constitué d'un groupe alkylène comprenant de 1 à 5 atomes de carbone.

11. Agent complexant selon l'une quelconque des revendications précédentes, **caractérisé en ce que** L₁, lorsqu'il est présent, est un groupe divalent de formule : 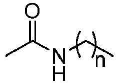 où n est un nombre entier de 1 à 16, de préférence de 1 à 5.

12. Agent complexant selon l'une quelconque des revendications précédentes, **caractérisé en ce que** L₂, lorsqu'il est présent, est un groupe divalent de formule : 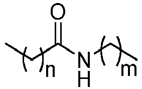 où n et m sont des nombres entiers de 1 à 16, de préférence de 1 à 5.

13. Agent complexant selon l'une quelconque des revendications précédentes, **caractérisé en ce que** L₃, lorsqu'il est présent, est un groupe divalent de formule : 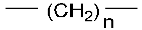 où n est un nombre entier de 1 à 16, de préférence de 1 à 5.

14. Complexe de lanthanide fluorescent comprenant un agent complexant selon l'une quelconque des revendications précédentes et un lanthanide choisi parmi : Eu³⁺, Sm³⁺, Tb³⁺.

15. Complexe de lanthanide fluorescent de formule (IV), (V), (VI) ou (VII) suivante : 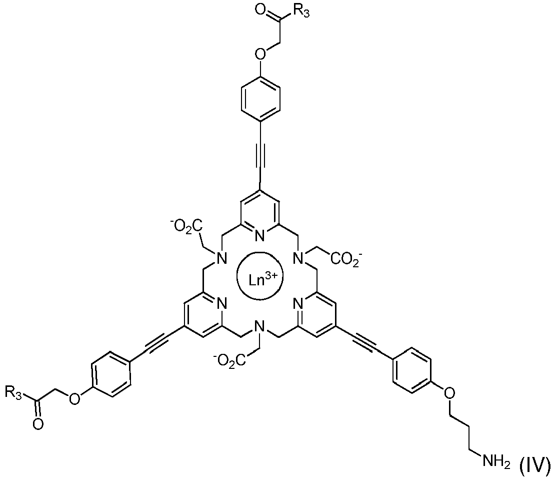 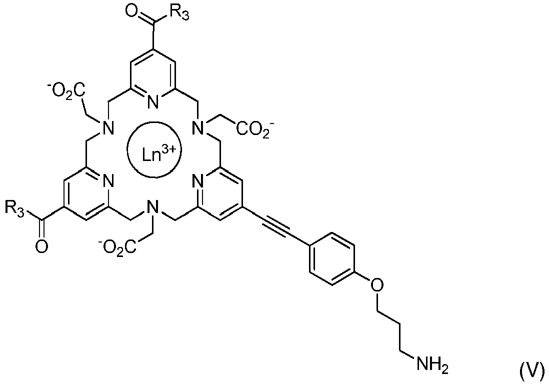 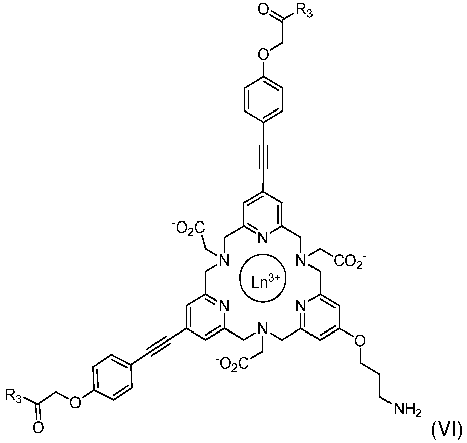 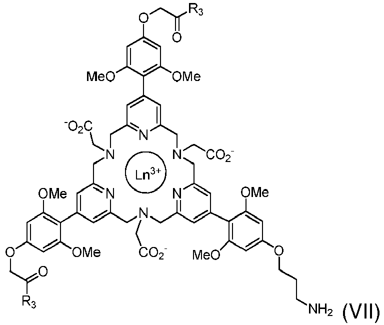 dans lesquelles :
Ln³⁺ est choisi parmi : Eu³⁺, Tb³⁺, Sm³⁺ ;
R₃ est choisi parmi les groupes suivants : OH ;

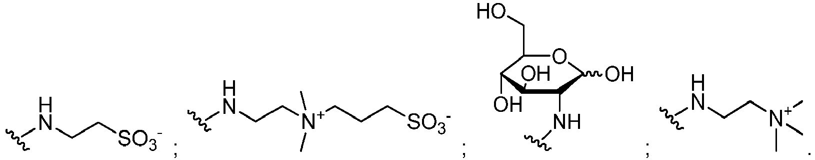

16. Conjugué fluorescent d'une molécule d'intérêt obtenu par la mise en contact d'un complexe de lanthanide selon les revendication 14 ou 15, ledit complexe comprenant un groupe réactif G, avec une molécule d'intérêt.

## Patentansprüche

1. Komplexbildner der Formel (I) 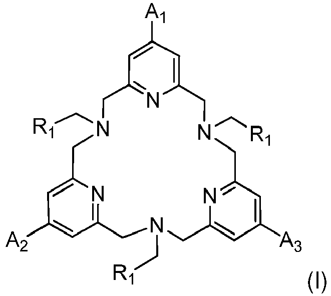 bei der:
- die Gruppen R₁ identisch sind und ausgewählt sind aus: -CO₂H, -PO(OH)R, wobei R ausgewählt ist aus den Gruppen: Phenyl, mit einer -SO₃H-Gruppe substituiertes Phenyl, vorzugsweise in ortho- oder para-Stellung, Benzyl, Methyl, Ethyl, Propyl, n-Butyl, sec-Butyl, Isobutyl, *tert*-Butyl,
- die Gruppen A₁, A₂ gleich oder verschieden sind und ausgewählt sind aus: einer Gruppe der Formel -L₁-E, einer Gruppe der Formel (II) oder (II'),
- die Gruppe A₃ ausgewählt ist aus: einer Gruppe der Formel -O-L₃-G, einer Gruppe der Formel (II), (II'), (III) oder (III'), 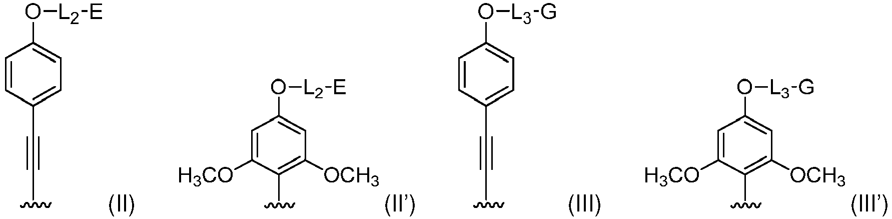 L₁, L₂ und L₃ identisch oder verschieden sind und eine zweiwertige Bindungsgruppe darstellen, die ausgewählt ist aus:
- einer linearen oder verzweigten C₁-C₂₀-Alkylengruppe, die eventuell eine oder mehrere Doppel- oder Dreifachbindungen enthält,
- einer C₅-C₈-Cycloalkylengruppe oder einer C₆-C₁₄-Arylengruppe,
wobei die Alkylen-, Cycloalkylen- oder Arylengruppen eventuell ein oder mehrere Heteroatome, wie Sauerstoff, Stickstoff, Schwefel, Phosphor oder eine oder mehrere Carbamoyl- oder Carboxamidogruppe(n) enthalten, und wobei die Alkylen-, Cycloalkylen- oder Arylengruppen eventuell mit C₁-C₈-Alkyl-, C₆-C₁₄-Aryl-, Sulfonat-oder Oxo-Gruppen substituiert sind,
- eine Gruppe, die aus den zweiwertigen Gruppen der folgenden Formeln ausgewählt ist: 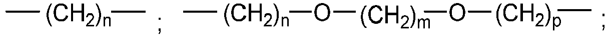 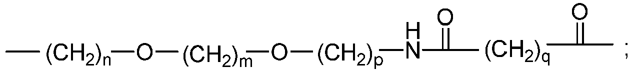 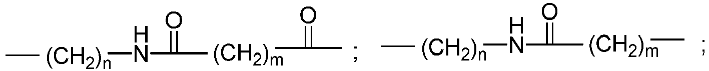 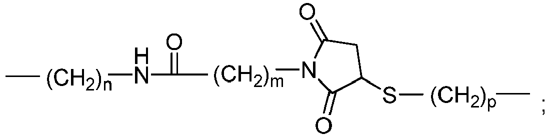 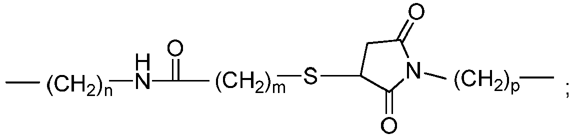 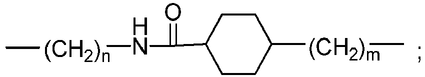 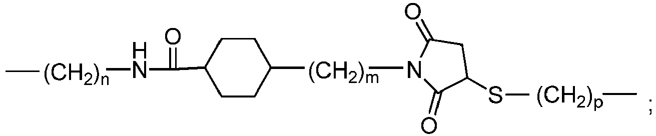 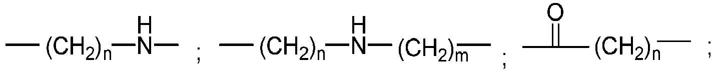 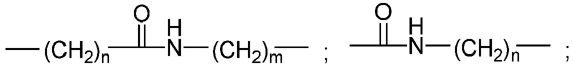 bei denen n, m, p, q ganze Zahlen von 1 bis 16, vorzugsweise von 1 bis 5 sind,
- E eine Gruppe ist, die den Komplexbildner wasserlöslich macht, ausgewählt aus: -SO₃H, -PO(OH)₂, -CO₂H, -N⁺Alk₁Alk₂Alk₃, einem Glucoserest in zyklischer oder linearer Form, oder aber einer Gruppe der Formel -(CHOH)ₖ-CH₂OH, wobei k eine ganze Zahl von 3 bis 12 ist; einem Sulfobetain; einer PEG-Gruppe der Formel -CH₂-(CH₂OCH₂)_{y}-CH₂OCH₃, wobei y eine ganze Zahl von 1 bis 5 ist,
- G eine reaktive Gruppe ist, die ausgewählt ist aus: einem Acrylamid, einem aktivierten Amin, einem aktivierten Ester, einem Aldehyd, ein Alkylhalogenid, einem Anhydrid, einem Anilin, einem Azid, ein Aziridin, einer Carbonsäure, einem Diazoalkan, einem Halogenacetamid, einem Halogentriazin, Dichlortriazin, einem Hydrazin, einem Imidoester, einem Isocyanat, einem Isothiocyanat, einem Maleimid, einem Sulfonylhalogenid, einem Thiol, einem Keton, einem Amin, einem Säurehalogenid, einem Succinimidylester, einem Hydroxysuccinimidylester, einem Hydroxysulfosuccinimidylester, einem Azidonitrophenyl, einem Azidophenyl, einem Glyoxal, einem Triazin, einer Acetylen-Gruppe, und den Gruppen der Formel: 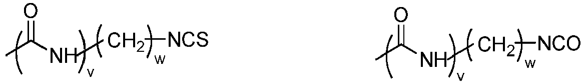 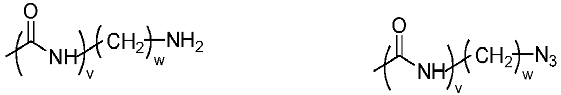 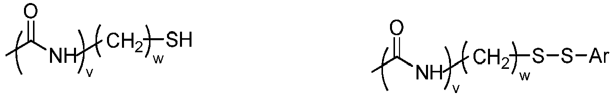 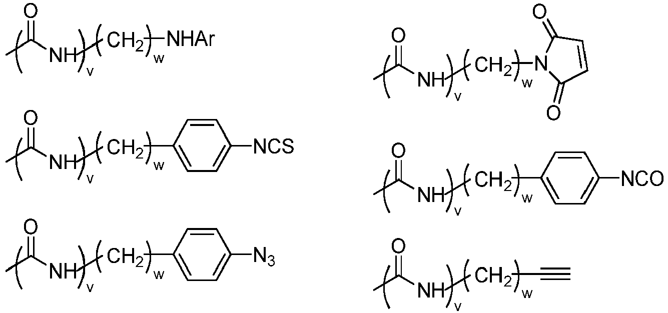 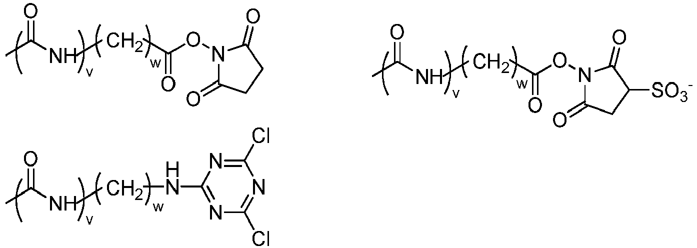 bei denen w von 0 bis 8 variiert und v gleich 0 oder 1 ist, und Ar ein gesättigter oder ungesättigter 5 oder 6-gliedriger Heterozyklus, mit 1 bis 3 Heteroatomen, eventuell mit einem Halogenatom substituiert, ist,
- Alk₁, Alk₂, Alk₃, die gleich oder verschieden sein können, ein (C₁-C₆)-Alkyl darstellen.

2. Komplexbildner nach Anspruch 1, **dadurch gekennzeichnet, dass** A₁ und A₂ identisch sind und eine Gruppe der Formel (II) sind und A₃ eine Gruppe der Formel (III) ist.

3. Komplexbildner nach Anspruch 1, **dadurch gekennzeichnet, dass** A₁ eine Gruppe der Formel (II) ist, A₂ eine Gruppe -L₁-E ist und A₃ eine Gruppe der Formel (III) ist.

4. Komplexbildner nach Anspruch 1, **dadurch gekennzeichnet, dass** A₁ und A₂ identisch sind und Gruppen der Formel (II) sind und A₃ eine Gruppe der Formel -O-L₃-G ist.

5. Komplexbildner nach Anspruch 1, **dadurch gekennzeichnet, dass** A₁ und A₂ identisch sind und Gruppen der Formel -L₁-E sind und A₃ eine Gruppe der Formel (III) ist.

6. Komplexbildner nach Anspruch 1, **dadurch gekennzeichnet, dass** A₁ und A₂ identisch sind und Gruppen der Formel (II') sind und A₃ eine Gruppe der Formel (III') ist.

7. Komplexbildner nach Anspruch 1, **dadurch gekennzeichnet, dass** A₁ eine Gruppe der Formel (II') ist, A₂ eine Gruppe -L-E ist und A₃ eine Gruppe der Formel (III') ist.

8. Komplexbildner nach Anspruch 1, **dadurch gekennzeichnet, dass** A₁ und A₂ identisch sind und Gruppen der Formel (II') sind und A₃ eine Gruppe der Formel -O-L₃-G ist.

9. Komplexbildner nach Anspruch 1, **dadurch gekennzeichnet, dass** A₁ und A₂ identisch sind und Gruppen der Formel -L₁-E sind und A₃ eine Gruppe der Formel (III') ist.

10. Komplexbildner nach einem der vorhergehenden Ansprüche, **dadurch gekennzeichnet, dass** die Gruppe -L₃-G, wenn vorhanden, aus einer reaktiven Gruppe G besteht, die ausgewählt ist aus: einer Carbonsäure, einem Amin, vorzugsweise einem aliphatischen Amin, einem Succinimidylester, einem Halogenacetamid, einem Hydrazin, einem Isothiocyanat, einer Maleimidgruppe, sowie aus einem L₃-Spacerarm, bestehend aus einer Alkylengruppe mit 1 bis 5 Kohlenstoffatomen

11. Komplexbildner nach einem der vorhergehenden Ansprüche, **dadurch gekennzeichnet, dass** L₁, wenn vorhanden, eine zweiwertige Gruppe ist der Formel: 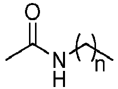 worin n eine ganze Zahl von 1 bis 16, vorzugsweise von 1 bis 5 ist.

12. Komplexbildner nach einem der vorhergehenden Ansprüche, **dadurch gekennzeichnet, dass** L₂, wenn vorhanden, eine zweiwertige Gruppe ist der Formel: 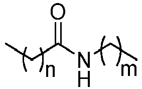 worin n und m ganze Zahlen von 1 bis 16, vorzugsweise von 1 bis 5 sind.

13. Komplexbildner nach einem der vorhergehenden Ansprüche, **dadurch gekennzeichnet, dass** L₃, wenn vorhanden, eine zweiwertige Gruppe ist der Formel: 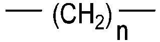 worin n eine ganze Zahl von 1 bis 16, vorzugsweise von 1 bis 5 ist.

14. Fluoreszierender Lanthanoid-Komplex, umfassend einen Komplexbildner nach einem der vorhergehenden Ansprüche und ein Lanthanoid, ausgewählt aus: Eu³⁺, Sm³⁺, Tb³⁺.

15. Fluoreszierender Lanthanoid-Komplex der folgenden Formel (IV), (V), (VI) oder (VII): 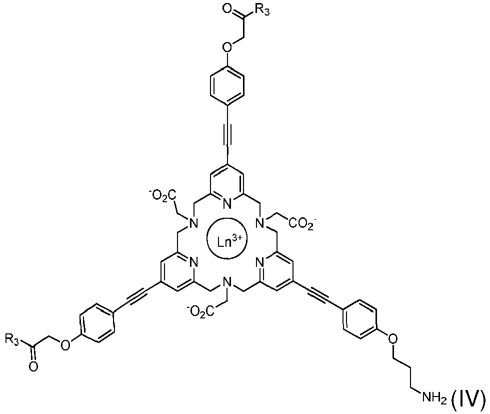 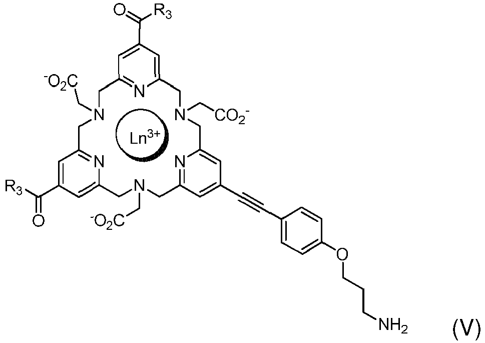 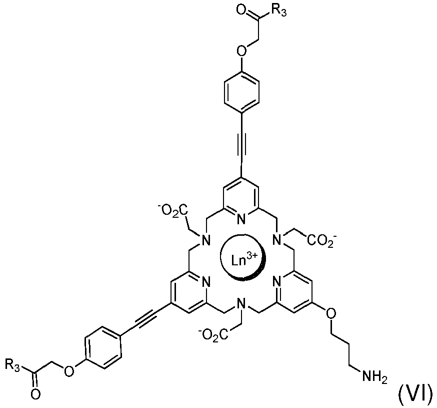 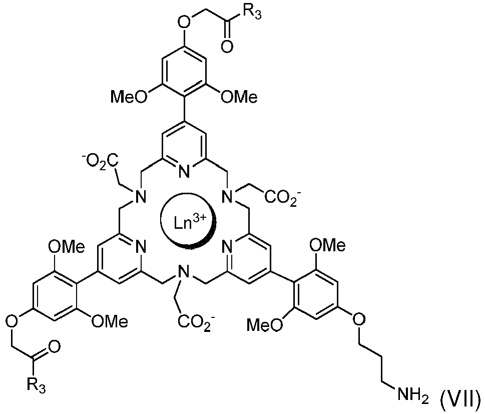 bei denen:
Ln³⁺ ausgewählt ist aus: Eu³⁺, Tb³⁺, Sm³⁺,
R₃ ausgewählt ist aus den folgenden Gruppen: OH,

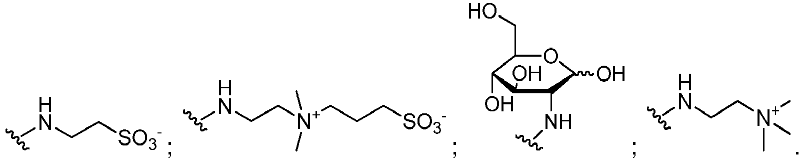

16. Fluoreszierendes Konjugat eines Moleküls von Interesse, erhalten durch das Kontaktieren eines Lanthanoid-Komplexes nach Anspruch 14 oder 15, wobei der Komplex eine reaktive Gruppe G umfasst, mit einem Molekül von Interesse.

## Claims

1. A complexing agent of formula (I): 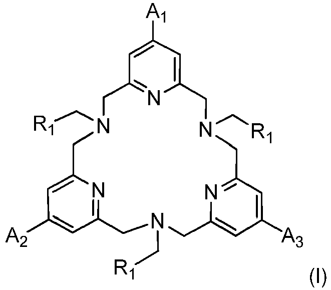 wherein:
- the R₁ groups are identical and are selected from: -CO₂H, -PO(OH)R, R being selected from the groups: phenyl, phenyl substituted by an -SO₃H group, preferably in the ortho or para position, benzyl, methyl, ethyl, propyl, n-butyl, sec-butyl, isobutyl, *tert*-butyl;
- the A₁, A₂ groups are identical or different and are selected from: a group of formula -L₁-E; a group of formula (II) or (II');
- the A₃ group is selected from: a group of formula -O-L₃-G, a group of formula (II), (II'), (III) or (III'); 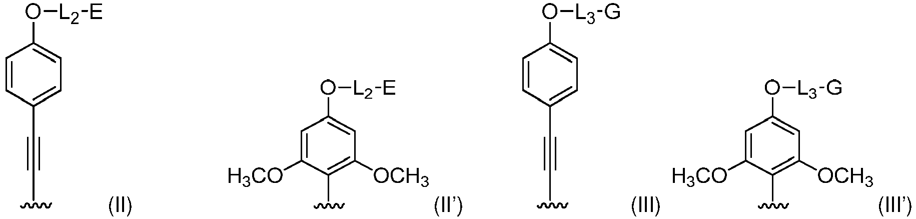
- L₁, L₂ and L₃ are identical or different and represent a divalent linkage group selected from:
- a linear or branched C₁-C₂₀ alkylene group optionally containing one or more double or triple bonds;
- a C₅-C₈ cycloalkylene group or a C₆-C₁₄ arylene group,
said alkylene, cycloalkylene or arylene groups optionally containing one or more hetero atoms, such as oxygen, nitrogen, sulfur, phosphorus or one or more carbamoyl or carboxamido groups, and said alkylene, cycloalkylene or arylene groups being optionally substituted by C₁-C₈ alkyl, C₆-C₁₄ aryl, sulfonate or oxo groups;
- a group chosen from the divalent groups of following formulae: 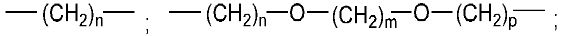 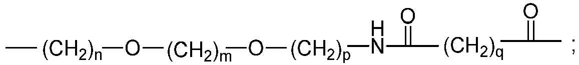 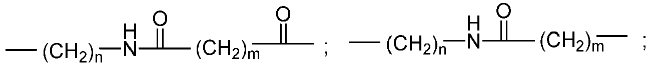 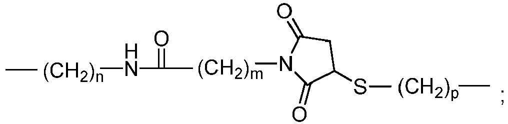 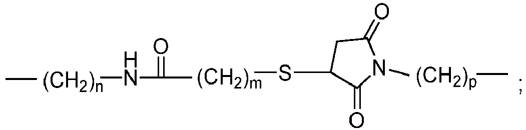 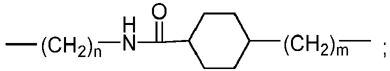 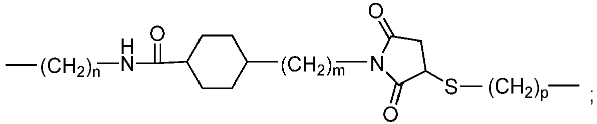 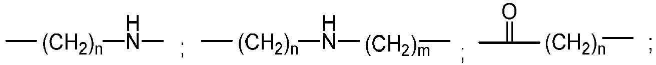 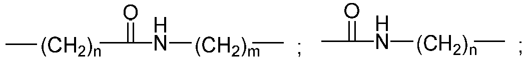 wherein n, m, p, q are integers from 1 to 16, preferably from 1 to 5;
- E is a group that renders the complexing agent water-soluble, selected from: -SO₃H; -PO(OH)₂; -CO₂H; -N⁺Alk₁Alk₂Alk₃; a glucose residue in cyclic or linear form, or else a group of formula -(CHOH)ₖ-CH₂OH, k being an integer ranging from 3 to 12; a sulfobetaine; a PEG group of formula -CH₂-(CH₂OCH₂)_{y}-CH₂OCH₃, y being an integer ranging from 1 to 5;
- G is a reactive group selected from: an acrylamide, an activated amine, an activated ester, an aldehyde, an alkyl halide, an anhydride, an aniline, an azide, an aziridine, a carboxylic acid, a diazoalkane, a haloacetamide, a halotriazine, dichlorotriazine, a hydrazine, an imido ester, an isocyanate, an isothiocyanate, a maleimide, a sulfonyl halide, a thiol, a ketone, an amine, an acid halide, a succinimidyl ester, a hydroxysuccinimidyl ester, a hydroxysulfosuccinimidyl ester, an azidonitrophenyl, an azidophenyl, a glyoxal, a triazine, an acetylenic group, and the groups of formula: 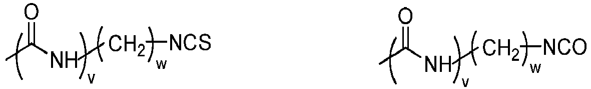 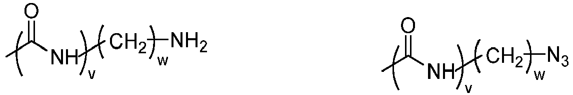 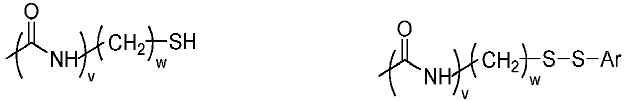 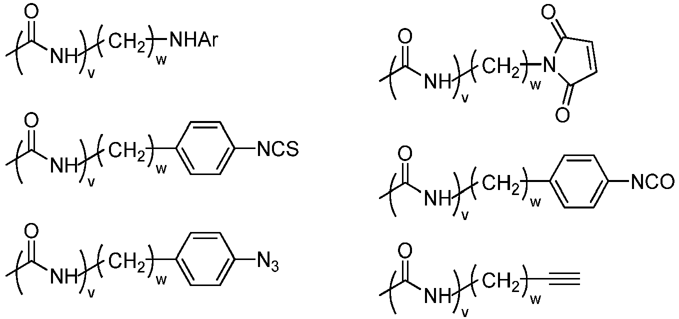 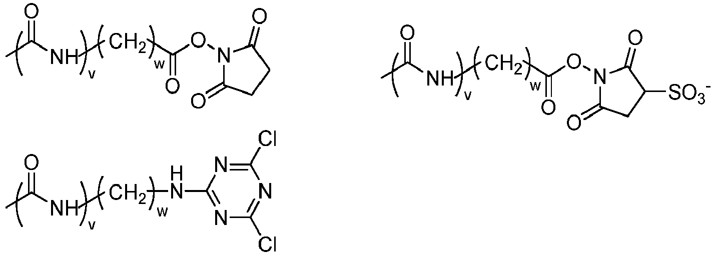 wherein w varies from 0 to 8 and v is equal to 0 or 1, and Ar is a saturated or unsaturated 5- or 6-membered heterocycle, comprising 1 to 3 heteroatoms, optionally substituted by a halogen atom;
- Alk₁, Alk₂, Alk₃, which may be identical or different, represent a (C₁-C₆)alkyl.

2. The complexing agent of claim 1, wherein A₁ and A₂ are identical and are a group of formula (II) and A₃ is a group of formula (III).

3. The complexing agent of claim 1, wherein A₁ is a group of formula (II), A₂ is an -L₁-E group and A₃ is a group of formula (III).

4. The complexing agent of claim 1, wherein A₁ and A₂ are identical and are groups of formula (II), and A₃ is a group of formula -O-L₃-G.

5. The complexing agent of claim 1, **characterized in that** A₁ and A₂ are identical and are groups of formula -L₁-E and A₃ is a group of formula (III).

6. The complexing agent as claimed in claim 1, wherein A₁ and A₂ are identical and are groups of formula (II') and A₃ is a group of formula (III').

7. The complexing agent of claim 1, wherein A₁ is a group of formula (II'), A₂ is an -L₁-E group and A₃ is a group of formula (III').

8. The complexing agent of claim 1, wherein A₁ and A₂ are identical and are groups of formula (II'), and A₃ is a group of formula -O-L₃-G.

9. The complexing agent of claim 1, wherein A₁ and A₂ are identical and are groups of formula - L₁-E and A₃ is a group of formula (III').

10. The complexing agent of any one of the preceding claims, wherein the -L₃-G group, when it is present, consists of a reactive group G selected from: a carboxylic acid, an amine, preferably an aliphatic amine, a succinimidyl ester, a haloacetamide, a hydrazine, an isothiocyanate, a maleimide group, and of a spacer arm L₃ consisting of an alkylene group comprising from 1 to 5 carbon atoms.

11. The complexing agent of any one of the preceding claims, wherein L₁, when it is present, is a divalent group of formula: 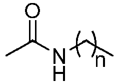 where n is an integer from 1 to 16, preferably from 1 to 5.

12. The complexing agent of any one of the preceding claims, wherein L₂, when it is present, is a divalent group of formula: 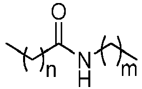 where n and m are integers from 1 to 16, preferably from 1 to 5.

13. The complexing agent of any one of the preceding claims, wherein L₃, when it is present, is a divalent group of formula: 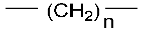 where n is an integer from 1 to 16, preferably from 1 to 5.

14. A fluorescent lanthanide complex comprising a complexing agent of any one of the preceding claims and a lanthanide selected from: Eu³⁺, Sm³⁺, Tb³⁺.

15. A fluorescent lanthanide complex of formula (IV), (V), (VI) or (VII) below: 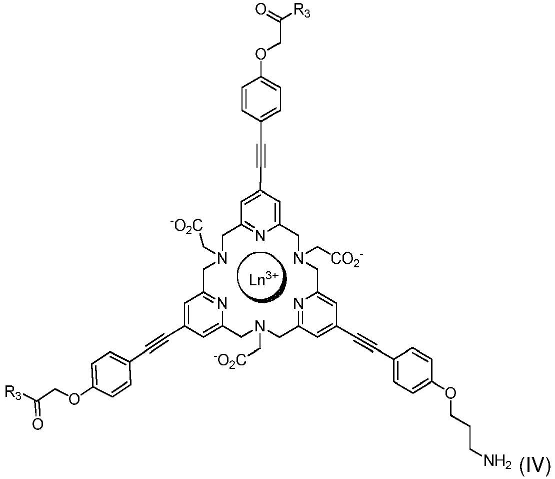 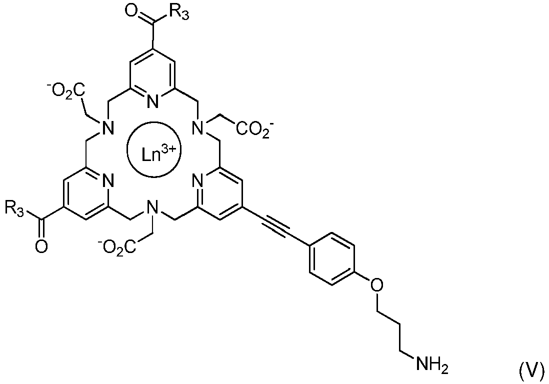 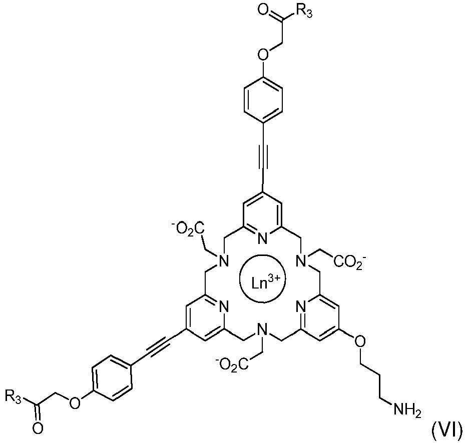 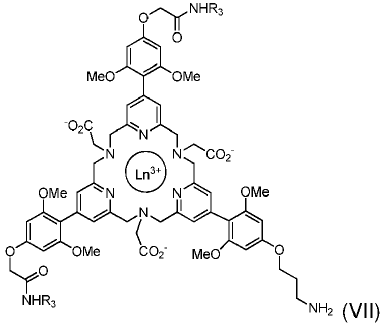 wherein:
Ln³⁺ is selected from: Eu³⁺, Tb³⁺, Sm³⁺;
R₃ is selected from the following groups: OH;

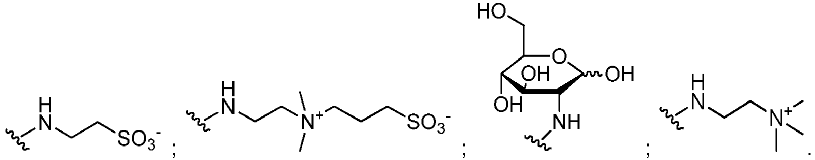

16. A fluorescent conjugate of a molecule of interest obtained by bringing a lanthanide complex of claim 14 or 15, said complex comprising a reactive group G, into contact with a molecule of interest.
